# EUROPEAN PATENT SPECIFICATION

(11) **EP 3 606 954 B1**
(45) Date of publication and mention of the grant of the patent: **20.07.2022**
(21) Application number: 18715026.3
(22) Date of filing: 03.04.2018
(51) Int. Cl.: C07K 16/28, A61K 39/395

(54) **ANTI-LAG3 ANTIBODIES**
ANTI-LAG3-ANTIKÖRPER
ANTICORPS ANTI-LAG3

(30) Priority: 05.04.2017 EP 17164917
(43) Date of publication of application: 12.02.2020
(73) Proprietor: F. Hoffmann-La Roche AG, 4070 Basel (CH)
(72) Inventor: CODARRI DEAK, Laura, 8952 Schlieren (CH); DENGL, Stefan, 82377 Penzberg (DE); FISCHER, Jens, 82377 Penzberg (DE); KLEIN, Christian, 8952 Schlieren (CH); SEEBER, Stefan, 82377 Penzberg (DE); WEBER, Patrick Alexander Aaron, 8952 Schlieren (CH); ZWICK, Adrian, 82377 Penzberg (DE)
(74) Representative: Jenni, Wolfgang
(86) International application number: PCT/EP2018/058385
(87) International publication number: WO 2018/185046

(56) References cited:
- WO-A1-2014/008218
- WO-A1-2015/138920
- WO-A1-2015/164665
- WO-A1-2016/079050
- WO-A1-2016/210129
- WO-A2-2010/019570
- DEMPKE WOLFRAM C M ET AL: "Second- and third-generation drugs for immuno-oncology treatment-The more the better?", EUROPEAN JOURNAL OF CANCER, ELSEVIER, AMSTERDAM, NL, vol. 74, 10 February 2017 (2017-02-10), pages 55-72, XP029933747, ISSN: 0959-8049, DOI: 10.1016/J.EJCA.2017.01.001

## Description

### FIELD OF THE INVENTION

The present invention relates to anti-LAG3 antibodies, to methods of producing these molecules and methods of using the same.

### BACKGROUND

Lymphocyte activation gene-3 (LAG3 or CD223) was initially discovered in an experiment designed to selectively isolate molecules expressed in an IL-2-dependent NK cell line (Triebel F et al., Cancer Lett. 235 (2006), 147-153). LAG-3 is a unique transmembrane protein with structural homology to CD4 with four extracellular immunoglobulin superfamily-like domains (D1-D4). The membrane-distal IgG domain contains a short amino acid sequence, the so-called extra loop that is not found in other IgG superfamily proteins. The intracellular domain contains a unique amino acid sequence (KIEELE) that is required for LAG-3 to exert a negative effect on T cell function. LAG-3 can be cleaved at the connecting peptide (CP) by metalloproteases to generate a soluble form, which is detectable in serum. Like CD4, the LAG3 protein binds to MHC class II molecules, however with a higher affinity and at a distinct site from CD4 (Huard et al. Proc. Natl. Acad. Sci. USA 94 (1997), 5744-5749). LAG3 is expressed by T cells, B cells, NK cells and plasmacytoid dendritic cells (pDCs) and is upregulated following T cell activation. It modulates T cell function as well as T cell homeostasis. Subsets of conventional T cells that are anergic or display impaired functions express LAG3. LAG3⁺ T cells are enriched at tumor sites and during chronic viral infections (Sierro et al Expert Opin. Ther. Targets 15 (2011), 91-101). It has been shown that LAG3 plays a role in CD8 T cell exhaustion (Blackburn et al. Nature Immunol. 10 (2009), 29-37). Thus, there is a need for antibodies that antagonize the activity of LAG3 and can be used to generate and restore immune response to tumors.

Monoclonal antibodies to LAG3 have been described, for example, in WO 2004/078928 wherein a composition comprising antibodies specifically binding to CD223 and an anti-cancer vaccine is claimed. WO 2010/019570 discloses human antibodies that bind LAG3, for example the antibodies 25F7 and 26H10. US 2011/070238 refers to a cytotoxic anti-LAG3 antibody useful in the treatment or prevention of organ transplant rejection and autoimmune disease. WO 2014/008218 describes LAG3 antibodies with optimized functional properties (i.e. reduced deamidation sites) compared to antibody 25F7. Furthermore, LAG3 antibodies are disclosed in WO 2015/138920 (for example BAP050), WO 2014/140180, WO 2015/116539, WO 2016/028672, WO 2016/126858, WO 2016/200782 and WO 2017/015560.

### SUMMARY

The invention is defined in the appended claims and any other aspects set forth herein not falling within the scope of the claims are for information only.

The invention provides an isolated antibody that binds to human LAG3, wherein the antibody comprises
(a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:4; (ii) HVR-L2 comprising the amino acid sequence of SEQ ID NO:5 and (iii) HVR-L3 comprising the amino acid sequence of SEQ ID NO:6.

The invention further provides an isolated antibody that binds to human LAG3, wherein the antibody
comprises a VH sequence of SEQ ID NO:7 and a VL sequence of SEQ ID NO:8.

In one embodiment of the invention such isolated antibody that binds to human LAG3:
i) competes for binding to LAG3 with an anti-LAG3 antibody comprising the VH with the amino acid sequence of SEQ ID NO:7 and VL with the amino acid sequence of SEQ ID NO:8, and/ or
ii) binds to a human and cynomolguoes LAG3; and/ or
iii) inhibits binding of MHC-II expressed on human A375 tumor cells; and/ or
iv) enhances granzyme B or IL-2 release in a mixed lymphocyte reaction (mMLR) assay.

In one embodiment the anti-LAG3 antibody according to the invention is a monoclonal antibody.

In one embodiment the anti-LAG3 antibody according to the invention is a human, humanized, or chimeric antibody.

The invention provides an isolated nucleic acid encoding such isolated antibody that binds to human LAG3.

The invention provides a host cell comprising such nucleic acid.

The invention provides a method of producing an antibody comprising culturing the host cell so that the antibody is produced.

The invention provides such method of producing an antibody, further comprising recovering the antibody from the host cell.

The invention provides a pharmaceutical formulation comprising the antibody described herein and a pharmaceutically acceptable carrier.

The invention provides the antibody described herein for use in treating cancer.

The antibodies of the present invention show valuable properties the induction of Granzyme B release, IFN-γ release and IL-2 secretion by human CD4 T cells and can therefore stimulate immune response via T cells (enhanced tumor-antigen specific T cell effector functions) either alone or in combination PD1 inhibitors.

### BRIEF DESCRIPTION OF THE FIGURES

- **Figure 1:**: Effect of anti-LAG-3 antibodies on on cytotoxic Granzyme B release and IL-2 secretion by human CD4 T cells cocultured with allogeneic mature dendritic cells Fig 1A: Granzyme B secretion Fig 1B: IL-2 secretion
- **Figure 2:**: Effect of anti-LAG-3 antibodies on cytotoxic Granzyme B release by human CD4 T cells cocultured with a B cell-lymphoblatoid cell line (ARH77).
- **Figure 3:**: Effect of anti-LAG-3 antibodies on Treg suppression of Granzyme B and IFN-γ release by human CD4 T cells cocultured with irradiated allogeneic PBMCs.
- Fig 3A:: Granzyme B release
- Fig 3B:: IFN-γ release

### DETAILED DESCRIPTION OF THE INVENTION

The invention is defined in the appended claims and any other aspects set forth herein not falling within the scope of the claims are for information only.

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The term "LAG3", as used herein, refers to any native LAG3 from any vertebrate source, including mammals such as primates (e.g. humans) and rodents (e.g., mice and rats), unless otherwise indicated. The term encompasses "full-length," unprocessed LAG3 as well as any form of LAG3 that results from processing in the cell. The term also encompasses naturally occurring variants of LAG3, e.g., splice variants or allelic variants. In one preferred embodiment the term "LAG3," refers to human LAG3. The amino acid sequence of an exemplary processed (without signal sequences) LAG3 is shown in SEQ ID NO: 54. The amino acid sequence of an exemplary Extracellular Domaisn (ECD) LAG3 is shown in SEQ ID NO: 55.

The terms "anti-LAG3 antibody" and "an antibody that binds to LAG3" refer to an antibody that is capable of binding LAG3 with sufficient affinity such that the antibody is useful as a diagnostic and/or therapeutic agent in targeting LAG3. In one embodiment, the extent of binding of an anti-LAG3 antibody to an unrelated, non-LAG3 protein is less than about 10% of the binding of the antibody to LAG3 as measured, e.g., by a radioimmunoassay (RIA). In certain embodiments, an antibody that binds to LAG3 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M). In certain embodiments, an anti-LAG3 antibody binds to an epitope of LAG3 that is conserved among LAG3 from different species. In one preferred embodiment, an "anti-LAG3 antibody", "an antibody that specifically binds to human LAG3", and "an antibody that binds to human LAG3" refers to an antibody specifically binding to the human LAG3 antigen or its Extracellular Domain (ECD) with a binding affinity of a K_{D}-value of 1.0 × 10⁻⁸ mol/l or lower, in one embodiment of a K_{D}-value of 1.0 × 10⁻⁹ mol/l or lower, in one embodiment of a K_{D}-value of 1.0 × 10⁻⁹ mol/l to 1.0 × 10⁻¹³ mol/l. In this context the binding affinity is determined with a standard binding assay, such as surface plasmon resonance technique (BIAcore^{®}, GE-Healthcare Uppsala, Sweden) e.g. using the LAG3 extracellular domain.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.

The term "epitope" denotes the site on an antigen, either proteinaceous or non-proteinaceous, to which an anti-LAG3 antibody binds. Epitopes can be formed both from contiguous amino acid stretches (linear epitope) or comprise non-contiguous amino acids (conformational epitope), e.g. coming in spatial proximity due to the folding of the antigen, i.e. by the tertiary folding of a proteinaceous antigen. Linear epitopes are typically still bound by an anti-LAG3 antibody after exposure of the proteinaceous antigen to denaturing agents, whereas conformational epitopes are typically destroyed upon treatment with denaturing agents. An epitope comprises at least 3, at least 4, at least 5, at least 6, at least 7, or 8-10 amino acids in a unique spatial conformation.

Screening for antibodies binding to a particular epitope (i.e., those binding to the same epitope) can be done using methods routine in the art such as, e.g., without limitation alanine scanning, peptide blots (see Meth. Mol. Biol. 248 (2004) 443-463), peptide cleavage analysis, epitope excision, epitope extraction, chemical modification of antigens (see Prot. Sci. 9 (2000) 487-496), and cross-blocking (see "Antibodies," Harlow and Lane (Cold Spring Harbor Press, Cold Spring Harb., NY).

Antigen Structure-based Antibody Profiling (ASAP), also known as Modification-Assisted Profiling (MAP), allows to bin a multitude of monoclonal antibodies specifically binding to LAG3 based on the binding profile of each of the antibody from the multitude to chemically or enzymatically modified antigen surfaces (see, e.g., US 2004/0101920). The antibodies in each bin bind to the same epitope which may be a unique epitope either distinctly different from or partially overlapping with epitope represented by another bin.

Also competitive binding can be used to easily determine whether an antibody binds to the same epitope of LAG3 as, or competes for binding with, a reference anti-LAG3 antibody. For example, an "antibody that binds to the same epitope" as a reference anti-LAG3 antibody refers to an antibody that blocks binding of the reference anti-LAG3 antibody to its antigen in a competition assay by 50% or more, and conversely, the reference antibody blocks binding of the antibody to its antigen in a competition assay by 50% or more. Also for example, to determine if an antibody binds to the same epitope as a reference anti-LAG3 antibody, the reference antibody is allowed to bind to LAG3 under saturating conditions. After removal of the excess of the reference anti-LAG3 antibody, the ability of an anti-LAG3 antibody in question to bind to LAG3 is assessed. If the anti-LAG3 antibody is able to bind to LAG3 after saturation binding of the reference anti-LAG3 antibody, it can be concluded that the anti-LAG3 antibody in question binds to a different epitope than the reference anti-LAG3 antibody. But, if the anti-LAG3 antibody in question is not able to bind to LAG3 after saturation binding of the reference anti-LAG3 antibody, then the anti-LAG3 antibody in question may bind to the same epitope as the epitope bound by the reference anti-LAG3 antibody. To confirm whether the antibody in question binds to the same epitope or is just hampered from binding by steric reasons routine experimentation can be used (e.g., peptide mutation and binding analyses using ELISA, RIA, surface plasmon resonance, flow cytometry or any other quantitative or qualitative antibody-binding assay available in the art). This assay should be carried out in two set-ups, i.e. with both of the antibodies being the saturating antibody. If, in both set-ups, only the first (saturating) antibody is capable of binding to LAG3, then it can be concluded that the anti-LAG3 antibody in question and the reference anti-LAG3 antibody compete for binding to LAG3.

In some embodiments two antibodies are deemed to bind to the same or an overlapping epitope if a 1-, 5-, 10-, 20- or 100-fold excess of one antibody inhibits binding of the other by at least 50%, at least 75%, at least 90% or even 99% or more as measured in a competitive binding assay (see, e.g., Junghans et al., Cancer Res. 50 (1990) 1495-1502).

In some embodiments two antibodies are deemed to bind to the same epitope if essentially all amino acid mutations in the antigen that reduce or eliminate binding of one antibody also reduce or eliminate binding of the other. Two antibodies are deemed to have "overlapping epitopes" if only a subset of the amino acid mutations that reduce or eliminate binding of one antibody reduce or eliminate binding of the other.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgAi, and IgA₂. In certain embodiments, the antibody is of the IgG4 isotype with the S228P mutation in the hinge region to improve stability of IgG4 antibody. The heavy chain constant domains that correspond to the different classes of immunoglobulins are called α, δ, ε, γ, and µ, respectively.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. In one embodiment the anti-Lag3 antibody as described herein is of IgG1 isotype and comprises an constant heavy chain domain of SEQ ID NO: 51 or of SEQ ID NO: 52. In one embodiment it comprises addtionall the C-terminal lysine (Lys447). In one embodiment the anti-Lag3 antibody as described herein is of IgG4 isotype and comprises an constant heavy chain domain of SEQ ID NO: 53. In one embodiment it comprises addtionall the C-terminal lysine (Lys447). Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. In certain embodiments, a human antibody is derived from a non-human transgenic mammal, for example a mouse, a rat, or a rabbit. In certain embodiments, a human antibody is derived from a hybridoma cell line.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (HI, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (HI), 26-35b (HI), 49-65 (H2), 93-102 (H3), and 94-102 (H3).

In one embodiment, HVR residues comprise those identified in the Description of the amino acid sequences below.

Unless otherwise indicated, HVR residues and other residues in the variable domain (e.g., FR residues) are numbered herein according to Kabat et al., *supra.*

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-LAG3 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CHI, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity. Alignment for purposes of determining percent amino acid sequence identity can be achieved in various ways that are within the skill in the art, for instance, using publicly available computer software such as BLAST, BLAST-2, Clustal W, Megalign (DNASTAR) software or the FASTA program package. Those skilled in the art can determine appropriate parameters for aligning sequences, including any algorithms needed to achieve maximal alignment over the full length of the sequences being compared. For purposes herein, however, % amino acid sequence identity values are generated using the ggsearch program of the FASTA package version 36.3.8c or later with a BLOSUM50 comparison matrix. The FASTA program package was authored by W. R. Pearson and D. J. Lipman (1988), "Improved Tools for Biological Sequence Analysis", PNAS 85:2444-2448; W. R. Pearson (1996) "Effective protein sequence comparison" Meth. Enzymol. 266:227- 258; and Pearson et. al. (1997) Genomics 46:24-36 and is publicly available from http://fasta.bioch.virginia.edu/fasta_www2/fasta_down.shtml. Alternatively, a public server accessible at http://fasta.bioch.virginia.edu/fasta_www2/index.cgi can be used to compare the sequences, using the ggsearch (global protein:protein) program and default options (BLOSUM50; open: -10; ext: -2; Ktup = 2) to ensure a global, rather than local, alignment is performed. Percent amino acid identity is given in the output alignment header.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

### I. COMPOSITIONS AND METHODS

In one aspect, the invention provides isolated antibodies that binds to LAG3.

In certain embodiments, antibodies that bind to human LAG3 are provided. Antibodies of the invention are useful, e.g., for the diagnosis or treatment of cancer, for treating or delaying progression of an immune related disease such as tumor immunity, or for stimulating an immune response or function, such as T cell activity; or for use as immunostimmulatory agent/ or stimulating granzyme B (GrzB), interferon-gamma (IFN-gamma) and or interleukin 2 (IL-2) secretion/release.

### A. Exemplary Anti-LAG3 Antibodies

In certain embodiments, an anti-LAG3 is provided wherein the antibody:
i) competes for binding to LAG3 with an anti-LAG3 antibody (comprising the VH and VL of aLAG3(0414)) comprising the VH with the amino acid sequence of SEQ ID NO:7 and VL with the amino acid sequence of SEQ ID NO:8, and/ or
ii) binds to a human and cynomolguoes LAG3; and/ or
iii) inhibits binding of MHC-II expressed on human A375 tumor cells; and/ or
iv) enhances granzyme B or IL-2 release in a mixed lymphocyte reaction (mMLR) assay (as shown in Example 3).

In one aspect, the invention provides an anti-LAG3 antibody comprising six HVRs selected from (a) HVR-H1 comprising the amino acid sequence of SEQ ID NO: 1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:4; (e) HVR-L2 comprising the amino acid sequence of SEQ ID NO:5; and (f) HVR-L3 comprising the amino acid sequence of SEQ ID NO:6.

In another aspect, an antibody of the invention comprises (a) a VH domain comprising all three VH HVR sequences selected from (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising all three VL HVR sequences selected from (i) HVR-L1 comprising the amino acid sequence of SEQ ID NO:4, (ii) HVR-L2 comprising the amino acid sequence of SEQ ID NO:5, and (c) HVR-L3 comprising the amino acid sequence of SEQ ID NO:6.

In another aspect, the invention provides an antibody comprising (a) HVR-H1 comprising the amino acid sequence of SEQ ID NO:1; (b) HVR-H2 comprising the amino acid sequence of SEQ ID NO:2; (c) HVR-H3 comprising the amino acid sequence of SEQ ID NO:3; (d) HVR-L1 comprising the amino acid sequence of SEQ ID NO:4; (e) HVR-L2 comprising the amino acid sequence of SEQ ID NO:5; and (f) HVR-L3 comprising an amino acid sequence selected from SEQ ID NO:6.

In any of the above embodiments, an anti-LAG3 antibody is human or humanized. In one embodiment, an anti-LAG3 antibody comprises HVRs as in any of the above embodiments, and further comprises an acceptor human framework, e.g. a human immunoglobulin framework or a human consensus framework.

In one embodiment, the antibody comprises the VH and VL sequences in SEQ ID NO:7 and SEQ ID NO:8, respectively, including post-translational modifications of those sequences.

In a further aspect of the invention, an anti-LAG3 antibody according to any of the above embodiments is a monoclonal antibody, including a chimeric, humanized or human antibody. In one embodiment, an anti-LAG3 antibody is an antibody fragment, e.g., a Fv, Fab, Fab', scFv, diabody, or F(ab')₂ fragment. In another embodiment, the antibody is a full length antibody, e.g., with the substitutions are L234A, L235A and P329G (LALA-PG) in an Fc region derived from a human IgG1 Fc region. (See, e.g., WO 2012/130831 Al).

In a further aspect, an anti-LAG3 antibody according to any of the above embodiments may incorporate any of the features, singly or in combination, as described in Sections 1-7 below:

### 1. Antibody Affinity

In certain embodiments, an antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20^{®}) in PBS. When the plates have dried, 150 µl/well of scintillant (MICROSCINT-20 ^{™}; Packard) is added, and the plates are counted on a TOPCOUNT^{™} gamma counter (Packard) for ten minutes. Concentrations of each Fab that give less than or equal to 20% of maximal binding are chosen for use in competitive binding assays.

According to another embodiment, Kd is measured using a BIACORE^{®} surface plasmon resonance assay. For example, an assay using a BIACORE^{®}-2000 or a BIACORE^{®}-3000 (BIAcore, Inc., Piscataway, NJ) is performed at 25°C with immobilized antigen CM5 chips at ~10 response units (RU). In one embodiment, carboxymethylated dextran biosensor chips (CM5, BIACORE, Inc.) are activated with *N*-ethyl-*N*'- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 µg/ml (~0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20^{™}) surfactant (PBST) at 25°C at a flow rate of approximately 25 µl/min. Association rates (kon) and dissociation rates (koff) are calculated using a simple one-to-one Langmuir binding model (BIACORE ^{®} Evaluation Software version 3.2) by simultaneously fitting the association and dissociation sensorgrams. The equilibrium dissociation constant (Kd) is calculated as the ratio koff/kon. See, e.g., Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25oC of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO^{™} spectrophotometer (ThermoSpectronic) with a stirred cuvette.

### 2. Antibody Fragments

In certain embodiments, an antibody provided herein is an antibody fragment. The term "antibody fragment" refers to a molecule other than an intact antibody that comprises a portion of an intact antibody that retains the ability to specifically bind to an antigen. Antibody fragments include, but are not limited to Fab, Fab', Fab'-SH, F(ab')₂, Fv, single-chain Fab (scFab); single-chain variable fragments (scFv) and single domain antibodies (dAbs). For a review of certain antibody fragments, see Holliger and Hudson, Nature Biotechnology 23:1126-1136 (2005).

In one embodiment, the antibody fragment is a Fab, Fab', Fab'-SH, or F(ab')₂ fragment, in particular a Fab fragment. Papain digestion of intact antibodies produces two identical antigen-binding fragments, called "Fab" fragments containing each the heavy- and light-chain variable domains and also the constant domain of the light chain and the first constant domain (CH1) of the heavy chain. The term "Fab fragment" thus refers to an antibody fragment comprising a light chain fragment comprising a VL domain and a constant domain of a light chain (CL), and a VH domain and a first constant domain (CH1) of a heavy chain. Fab' fragments differ from Fab fragments by the addition of residues at the carboxy terminus of the heavy chain CHI domain including one or more cysteines from the antibody hinge region. Fab'-SH are Fab' fragments in which the cysteine residue(s) of the constant domains bear a free thiol group. Pepsin treatment yields an F(ab')₂ fragment that has two antigen-combining sites (two Fab fragments) and a part of the Fc region. For discussion of Fab and F(ab')₂ fragments comprising salvage receptor binding epitope residues and having increased in vivo half-life, see U.S. Patent No. 5,869,046.

In another embodiment, the antibody fragment is a diabody, a triabody or a tetrabody. Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

In a further embodiment, the antibody fragment is a single chain Fab fragment. A "single chain Fab fragment" or "scFab" is a polypeptide consisting of an antibody heavy chain variable domain (VH), an antibody constant domain 1 (CH1), an antibody light chain variable domain (VL), an antibody light chain constant domain (CL) and a linker, wherein said antibody domains and said linker have one of the following orders in N-terminal to C-terminal direction: a) VH-CH1-linker-VL-CL, b) VL-CL-linker-VH-CH1, c) VH-CL-linker-VL-CH1 or d) VL-CH1-linker-VH-CL. In particular, said linker is a polypeptide of at least 30 amino acids, preferably between 32 and 50 amino acids. Said single chain Fab fragments are stabilized via the natural disulfide bond between the CL domain and the CHI domain. In addition, these single chain Fab molecules might be further stabilized by generation of interchain disulfide bonds via insertion of cysteine residues (e.g. position 44 in the variable heavy chain and position 100 in the variable light chain according to Kabat numbering).

In another embodiment, the antibody fragment is single-chain variable fragment (scFv). A "single-chain variable fragment (scFv)" is a fusion protein of the variable regions of the heavy (V_{H}) and light chains (V_{L}) of an antibody, connected by a linker. In particular, the linker is a short polypeptide of 10 to 25 amino acids and is usually rich in glycine for flexibility, as well as serine or threonine for solubility, and can either connect the N-terminus of the V_{H} with the C-terminus of the V_{L}, or *vice versa.* This protein retains the specificity of the original antibody, despite removal of the constant regions and the introduction of the linker. For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458.

In another embodiment, the antibody fragment is a single domain antibody. Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., U.S. Patent No. 6,248,516 B1).

Antibody fragments can be made by various techniques, including but not limited to proteolytic digestion of an intact antibody as well as production by recombinant host cells (e.g. E. coli or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE^{™} technology; U.S. Patent No. 5,770,429 describing HUMAB^{®} technology; U.S. Patent No. 7,041,870 describing K-M MOUSE^{®} technology, and U.S. Patent Application Publication No. US 2007/0061900, describing VELOCIMOUSE^{®} technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. Methods for screening combinatorial libraries are reviewed, e.g., in Lerner et al. in Nature Reviews 16:498-508 (2016). For example, a variety of methods are known in the art for generating phage display libraries and screening such libraries for antibodies possessing the desired binding characteristics. Such methods are reviewed, e.g., in Frenzel et al. in mAbs 8:1177-1194 (2016); Bazan et al. in Human Vaccines and Immunotherapeutics 8:1817-1828 (2012) and Zhao et al. in Critical Reviews in Biotechnology 36:276-289 (2016) as well as in Hoogenboom et al. in Methods in Molecular Biology 178:1-37 (O'Brien et al., ed., Human Press, Totowa, NJ, 2001) and in Marks and Bradbury in Methods in Molecular Biology 248:161-175 (Lo, ed., Human Press, Totowa, NJ, 2003).

In certain phage display methods, repertoires of VH and VL genes are separately cloned by polymerase chain reaction (PCR) and recombined randomly in phage libraries, which can then be screened for antigen-binding phage as described in Winter et al. in Annual Review of Immunology 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al. in EMBO Journal 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter in Journal of Molecular Biology 227: 381-388 (1992). Patent publications describing human antibody phage libraries include, for example: US Patent Nos. 5,750,373; 7,985,840; 7,785,903 and 8,679,490 as well as US Patent Publication Nos. 2005/0079574, 2007/0117126, 2007/0237764 and 2007/0292936.

Further examples of methods known in the art for screening combinatorial libraries for antibodies with a desired activity or activities include ribosome and mRNA display, as well as methods for antibody display and selection on bacteria, mammalian cells, insect cells or yeast cells. Methods for yeast surface display are reviewed, e.g., in Scholler et al. inMethods in Molecular Biology 503:135-56 (2012) and in Cherf et al. in Methods in Molecular biology 1319:155-175 (2015) as well as in the Zhao et al. in Methods in Molecular Biology 889:73-84 (2012). Methods for ribosome display are described, e.g., in He et al. in Nucleic Acids Research 25:5132-5134 (1997) and in Hanes et al. in PNAS 94:4937-4942 (1997).

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites, i.e., different epitopes on different antigens or different epitopes on the same antigen. In certain embodiments, the multispecific antibody has three or more binding specificities. In certain embodiments, one of the binding specificities is for LAG3 and the other (two or more) specificity is for any other antigen. In certain embodiments, bispecific antibodies may bind to two (or more) different epitopes of LAG3. Multispecific (e.g., bispecific) antibodies may also be used to localize cytotoxic agents or cells to cells which express LAG3. Multispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)) and "knob-in-hole" engineering (see, e.g., U.S. Patent No. 5,731,168, and Atwell et al., J. Mol. Biol. 270:26 (1997)). Multi-specific antibodies may also be made by engineering electrostatic steering effects for making antibody Fc-heterodimeric molecules (see, e.g., WO 2009/089004); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992) and WO 2011/034605); using the common light chain technology for circumventing the light chain mis-pairing problem (see, e.g., WO 98/50431); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more antigen binding sites, including for example, "Octopus antibodies," or DVD-Ig are also included herein (see, e.g. WO 2001/77342 and WO 2008/024715). Other examples of multispecific antibodies with three or more antigen binding sites can be found in WO 2010/115589, WO 2010/112193, WO 2010/136172, WO2010/145792, and WO 2013/026831. The bispecific antibody or antigen binding fragment thereof also includes a "Dual Acting FAb" or "DAF" comprising an antigen binding site that binds to LAG3 as well as another different antigen, or two different epitopes of LAG3 (see, e.g., US 2008/0069820 and WO 2015/095539).

Multi-specific antibodies may also be provided in an asymmetric form with a domain crossover in one or more binding arms of the same antigen specificity, i.e. by exchanging the VH/VL domains (see e.g., WO 2009/080252 and WO 2015/150447), the CH1/CL domains (see e.g., WO 2009/080253) or the complete Fab arms (see e.g., WO 2009/080251, WO 2016/016299, also see Schaefer et al, PNAS, 108 (2011) 1187-1191, and Klein at al., MAbs 8 (2016) 1010-20). In one embodiment, the multispecific antibody comprises a cross-Fab fragment. The term "**cross-Fab fragment**" or "xFab fragment" or "crossover Fab fragment" refers to a Fab fragment, wherein either the variable regions or the constant regions of the heavy and light chain are exchanged. A cross-Fab fragment comprises a polypeptide chain composed of the light chain variable region (VL) and the heavy chain constant region (CH1), and a polypeptide chain composed of the heavy chain variable region (VH) and the light chain constant region (CL). Asymmetrical Fab arms can also be engineered by introducing charged or non-charged amino acid mutations into domain interfaces to direct correct Fab pairing. See e.g., WO 2016/172485.

Various further molecular formats for multispecific antibodies are known in the art and are included herein (see e.g., Spiess et al., Mol Immunol 67 (2015) 95-106).

### 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g*. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the oligosaccharide attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a non-fucosylated oligosaccharide, i.e. an oligosaccharide structure that lacks fucose attached (directly or indirectly) to an Fc region. Such non-fucosylated oligosaccharide (also referred to as "afucosylated" oligosaccharide) particularly is an N-linked oligosaccharide which lacks a fucose residue attached to the first GlcNAc in the stem of the biantennary oligosaccharide structure. In one embodiment, antibody variants are provided having an increased proportion of non-fucosylated oligosaccharides in the Fc region as compared to a native or parent antibody. For example, the proportion of non-fucosylated oligosaccharides may be at least about 20%, at least about 40%, at least about 60%, at least about 80%, or even about 100% (i.e. no fucosylated oligosaccharides are present). The percentage of non-fucosylated oligosaccharides is the (average) amount of oligosaccharides lacking fucose residues, relative to the sum of all oligosaccharides attached to Asn 297 (e. g. complex, hybrid and high mannose structures) as measured by MALDI-TOF mass spectrometry, as described in WO 2006/082515, for example. Asn297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. Such antibodies having an increased proportion of non-fucosylated oligosaccharides in the Fc region may have improved FcγRIIIa receptor binding and/or improved effector function, in particular improved ADCC function. See, e.g., US 2003/0157108; US 2004/0093621.

Examples of cell lines capable of producing antibodies with reduced fucosylation include Lec13 CHO cells deficient in protein fucosylation (Ripka et al. Arch. Biochem. Biophys. 249:533-545 (1986); US 2003/0157108; and WO 2004/056312, especially at Example 11), and knockout cell lines, such as alpha-1,6-fucosyltransferase gene, *FUT8,* knockout CHO cells (see, e.g., Yamane-Ohnuki et al. Biotech. Bioeng. 87:614-622 (2004); Kanda, Y. et al., Biotechnol. Bioeng., 94(4):680-688 (2006); and WO2003/085107), or cells with reduced or abolished activity of a GDP-fucose synthesis or transporter protein (see, e.g., US2004259150, US2005031613, US2004132140, US2004110282).

In a further embodiment, antibody variants are provided with bisected oligosaccharides, e.g., in which a biantennary oligosaccharide attached to the Fc region of the antibody is bisected by GlcNAc. Such antibody variants may have reduced fucosylation and/or improved ADCC function as described above. Examples of such antibody variants are described, e.g., in Umana et al., Nat Biotechnol 17, 176-180 (1999); Ferrara et al., Biotechn Bioeng 93, 851-861 (2006); WO 99/54342; WO 2004/065540, WO 2003/011878.

Antibody variants with at least one galactose residue in the oligosaccharide attached to the Fc region are also provided. Such antibody variants may have improved CDC function. Such antibody variants are described, e.g., in WO 1997/30087; WO 1998/58964; and WO 1999/22764.

### c) Fc region variants

In certain embodiments, one or more amino acid modifications may be introduced into the Fc region of an antibody provided herein, thereby generating an Fc region variant. The Fc region variant may comprise a human Fc region sequence (*e.g.,* a human IgG1, IgG2, IgG3 or IgG4 Fc region) comprising an amino acid modification (e.g. a substitution) at one or more amino acid positions.

In certain embodiments, the invention contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcgammaR binding (hence likely lacking ADCC activity), but retains FcRn binding ability. The primary cells for mediating ADCC, NK cells, express FcgammaRIII only, whereas monocytes express FcgammaRI, FcgammaRII and FcgammaRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI^{™} non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12): 1759-1769 (2006); WO 2013/120929 Al).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In certain embodiments, an antibody variant comprises an Fc region with one or more amino acid substitutions, which increase FcRn binding, e.g., substitutions at positions 252, and/or 254, and/or 256 of the Fc region (EU numbering of residues). In certain embodiments, the antibody variant comprises an Fc region with the amino acid substitutions at positions 252, 254, and 256. In one embodiment the substitutions are M252Y, S254T and T256E in an Fc region derived from a human IgG1 Fc-region.

In certain embodiments, an antibody variant comprises an Fc region with amino acid substitutions, which diminish FcyR binding, e.g., substitutions at positions 234 and 235 of the Fc region (EU numbering of residues). In one embodiment the substitutions are L234A and L235A (LALA). In certain embodiments, the antibody variant further comprises D265A and/or P329G in an Fc region derived from a human IgG1 Fc region. In one embodiment the substitutions are L234A, L235A and P329G (LALA-PG) in an Fc region derived from a human IgG1 Fc region. (See, e.g., WO 2012/130831 Al). In another embodiment, the substitutions are L234A, L235A and D265A (LALA-DA) in an Fc region derived from a human IgG1 Fc region.

In some embodiments, alterations are made in the Fc region that result in altered (*i.e.,* either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US 2005/0014934A1 (Hinton et al.). Those antibodies comprise an Fc region with one or more substitutions therein which improve binding of the Fc region to FcRn. Such Fc variants include those with substitutions at one or more of Fc region residues: 238, 252, 254, 256, 265, 272, 286, 303, 305, 307, 311, 312, 317, 340, 356, 360, 362, 376, 378, 380, 382, 413, 424 or 434, e.g., substitution of Fc region residue 434 (See, e.g., US Patent No. 7,371,826; Dall'Acqua, W.F., et al. J. Biol. Chem. 281 (2006) 23514-23524).

Fc region residues critical to the mouse Fc-mouse FcRn interaction have been identified by site-directed mutagenesis (see e.g. Dall'Acqua, W.F., et al. J. Immunol 169 (2002) 5171-5180). Residues 1253, H310, H433, N434, and H435 (EU numbering according to Kabat) are involved in the interaction (Medesan, C., et al., Eur. J. Immunol. 26 (1996) 2533; Firan, M., et al., Int. Immunol. 13 (2001) 993; Kim, J.K., et al., Eur. J. Immunol. 24 (1994) 542). Residues 1253, H310, and H435 were found to be critical for the interaction of human Fc with murine FcRn (Kim, J.K., et al., Eur. J. Immunol. 29 (1999) 2819). Studies of the human Fc-human FcRn complex have shown that residues 1253, S254, H435, and Y436 are crucial for the interaction (Firan, M., et al., Int. Immunol. 13 (2001) 993; Shields, R.L., et al., J. Biol. Chem. 276 (2001) 6591-6604). In Yeung, Y.A., et al. (J. Immunol. 182 (2009) 7667-7671) various mutants of residues 248 to 259 and 301 to 317 and 376 to 382 and 424 to 437 have been reported and examined.

In certain embodiments, an antibody variant comprises an Fc region with one or more amino acid substitutions, which reduce FcRn binding, e.g., substitutions at positions 253, and/or 310, and/or 435 of the Fc-region (EU numbering of residues). In certain embodiments, the antibody variant comprises an Fc region with the amino acid substitutions at positions 253, 310 and 435. In one embodiment the substitutions are I253A, H310A and H435A in an Fc region derived from a human IgG1 Fc-region. See e.g., Grevys, A., et al., J. Immunol. 194 (2015) 5497-5508.

In certain embodiments, an antibody variant comprises an Fc region with one or more amino acid substitutions, which reduce FcRn binding, e.g., substitutions at positions 310, and/or 433, and/or 436 of the Fc region (EU numbering of residues). In certain embodiments, the antibody variant comprises an Fc region with the amino acid substitutions at positions 310, 433 and 436. In one embodiment the substitutions are H310A, H433A and Y436A in an Fc region derived from a human IgG1 Fc-region. (See, e.g., WO 2014/177460 Al).

See also Duncan & Winter, Nature 322:738-40 (1988); U.S. Patent No. 5,648,260; U.S. Patent No. 5,624,821; and WO 94/29351 concerning other examples of Fc region variants.

### B. Recombinant Methods and Compositions

Antibodies may be produced using recombinant methods and compositions, e.g., as described in US 4,816,567. For these methods one or more isolated nucleic acid(s) encoding an antibody are provided.

In case of a native antibody or native antibody fragment two nucleic acids are required, one for the light chain or a fragment thereof and one for the heavy chain or a fragment thereof. Such nucleic acid(s) encode an amino acid sequence comprising the VL and/or an amino acid sequence comprising the VH of the antibody (e.g., the light and/or heavy chain(s) of the antibody). These nucleic acids can be on the same expression vector or on different expression vectors.

In case of a bispecific antibody with heterodimeric heavy chains four nucleic acids are required, one for the first light chain, one for the second light chain comprising the first hetreomonomeric Fc-region polypeptide, one for the second light chain, and one for the second heavy chain comprising the second heteromonomeric Fc-region polypeptide. The four nucleic acids can be comprised in one or more nucleic acid molecules or expression vectors. Such nucleic acid(s) encode an amino acid sequence comprising the first VL and/or an amino acid sequence comprising the first VH including the first heteromonomeric Fc-region and/or an amino acid sequence comprising the second VL and/or an amino acid sequence comprising the second VH including the second heteromonomeric Fc-region of the antibody (e.g., the first and/or second light and/or the first and/or second heavy chains of the antibody). These nucleic acids can be on the same expression vector or on different expression vectors, normally these nucleic acids are located on two or three expression vectors, i.e. one vector can comprise more than one of these nucleic acids. Examples of these bispecific antibodies are CrossMabs and T-cell bispecifics (see, e.g. Schaefer, W. et al, PNAS, 108 (2011) 11187-1191). For example, one of the heteromonomeric heavy chain comprises the so-called "knob mutations" (T366W and optionally one of S354C or Y349C) and the other comprises the so-called "hole mutations" (T366S, L368A and Y407V and optionally Y349C or S354C) (see, e.g., Carter, P. et al., Immunotechnol. 2 (1996) 73).

In one embodiment isolated nucleic acids encoding an antibody as used in the methods as reported herein are provided.

In a further embodiment, one or more vectors (e.g., expression vectors) comprising such nucleic acid(s) are provided.

In a further embodiment, a host cell comprising such nucleic acid(s) is provided.

In one such embodiment, a host cell comprises (e.g., has been transformed with):
- in case of an antibody composed of two identical light chains and two identical heavy chains that are disulfide-bonded.or a VH and VL comprising fragment thereof:
   (1) a vector comprising nucleic acids that encode an amino acid sequence comprising the VL of the antibody and an amino acid sequence comprising the VH of the antibody, or
   (2) a first vector comprising a nucleic acid that encodes an amino acid sequence comprising the VL of the antibody and a second vector comprising a nucleic acid that encodes an amino acid sequence comprising the VH of the antibody.
- in case of a bispecific antibody with heterodimeric heavy chains:
   (1) a first vector comprising a first pair of nucleic acids that encode amino acid sequences one of them comprising the first VL and the other comprising the first VH of the antibody and a second vector comprising a second pair of nucleic acids that encode amino acid sequences one of them comprising the second VL and the other comprising the second VH of the antibody, or
   (2) a first vector comprising a first nucleic acid that encode an amino acid sequence comprising one of the variable domains (preferably a light chain variable domain), a second vector comprising a pair of nucleic acids that encode amino acid sequences one of them comprising a light chain variable domain and the other comprising the first heavy chain variable domain, and a third vector comprising a pair of nucleic acids that encode amino acid sequences one of them comprising the respective other light chain variable domain as in the second vector and the other comprising the second heavy chain variable domain, or
   (3) a first vector comprising a nucleic acid that encodes an amino acid sequence comprising the first VL of the antibody, a second vector comprising a nucleic acid that encodes an amino acid sequence comprising the first VH of the antibody, a third vector comprising a nucleic acid that encodes an amino acid sequence comprising the second VL of the antibody, and a fourth vector comprising a nucleic acid that encodes an amino acid sequence comprising the second VH of the antibody.

In one embodiment, the host cell is eukaryotic, e.g. a Chinese Hamster Ovary (CHO) cell or lymphoid cell (e.g., Y0, NS0, Sp20 cell). In one embodiment, a method of making an anti-LAG3 antibody is provided, wherein the method comprises culturing a host cell comprising nucleic acids encoding the antibody, as provided above, under conditions suitable for expression of the antibody, and optionally recovering the antibody from the host cell (or host cell culture medium).

For recombinant production of an anti-LAG3 antibody, nucleic acids encoding an antibody, e.g., as described above, are isolated and inserted into one or more vectors for further cloning and/or expression in a host cell. Such nucleic acids may be readily isolated and sequenced using conventional procedures (e.g., by using oligonucleotide probes that are capable of binding specifically to genes encoding the heavy and light chains of the antibody) or produced by recombinant methods or obtained by chemical synthesis.

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., US 5,648,237, US 5,789,199, and US 5,840,523. (See also Charlton, K.A., In: Methods in Molecular Biology, Vol. 248, Lo, B.K.C. (ed.), Humana Press, Totowa, NJ (2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, T.U., Nat. Biotech. 22 (2004) 1409-1414; and Li, H. et al., Nat. Biotech. 24 (2006) 210-215.

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. See, e.g., US 5,959,177, US 6,040,498, US 6,420,548, US 7,125,978, and US 6,417,429 (describing PLANTIBODIESTM technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P. et al., Annals N.Y. Acad. Sci. 383 (1982) 44-68; MRC 5 cells; and FS4 cells. Other useful mammalian host cell lines include Chinese hamster ovary (CHO) cells, including DHFR- CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### C. Assays

Anti-LAG3 antibodies provided herein may be identified, screened for, or characterized for their physical/chemical properties and/or biological activities by various assays known in the art.

### 1. Binding assays and other assays

In one aspect, an antibody of the invention is tested for its antigen binding activity, e.g., by known methods such as ELISA, Western blot, etc.

In another aspect, competition assays may be used to identify an antibody that competes with aLAG3(0414) for binding to LAG3. In certain embodiments, such a competing antibody binds to the same epitope (e.g., a linear or a conformational epitope) that is bound by aLAG3(0414). Detailed exemplary methods for mapping an epitope to which an antibody binds are provided in Morris (1996) "Epitope Mapping Protocols," in Methods in Molecular Biology vol. 66 (Humana Press, Totowa, NJ).

In an exemplary competition assay, immobilized LAG3 is incubated in a solution comprising a first labeled antibody that binds to LAG3 (e.g. aLAG3(0414)) and a second unlabeled antibody that is being tested for its ability to compete with the first antibody for binding to LAG3. The second antibody may be present in a hybridoma supernatant. As a control, immobilized LAG3 is incubated in a solution comprising the first labeled antibody but not the second unlabeled antibody. After incubation under conditions permissive for binding of the first antibody to LAG3, excess unbound antibody is removed, and the amount of label associated with immobilized LAG3 is measured. If the amount of label associated with immobilized LAG3 is substantially reduced in the test sample relative to the control sample, then that indicates that the second antibody is competing with the first antibody for binding to LAG3. See Harlow and Lane (1988) Antibodies: A Laboratory Manual ch. 14 (Cold Spring Harbor Laboratory, Cold Spring Harbor, NY).

### 2. Activity assays

In one aspect, assays are provided for identifying anti-LAG3 antibodies thereof having biological activity. Biological activity may include, e.g., effect of anti-LAG3 antibodies (alone or in combination with anti-PDL1 antibodies) on cytotoxic Granzyme B release and IL-2 secretion by human CD4 T cells in amixed lymphocyte reaction (mMLR) assa or the effect of anti-LAG-3 antibodies on Treg suppression of Granzyme B and IFN-γ release by human CD4 T cells; or the Inhibition of LAG-3 binding to MHC-II expressed on human A375 tumor cells by anti-LAG3 antibodies. Antibodies having such biological activity in vivo and/or in vitro are also provided.

In certain embodiments, an antibody of the invention is tested for such biological activity. For details, see examples 2 and 3 below.

### D. Methods and Compositions for Diagnostics and Detection

In certain embodiments, any of the anti-LAG3 antibodies provided herein is useful for detecting the presence of LAG3 in a biological sample. The term "detecting" as used herein encompasses quantitative or qualitative detection. In certain embodiments, a biological sample comprises a cell or tissue, such as tumor tissue.

In one embodiment, an anti-LAG3 antibody for use in a method of diagnosis or detection is provided. In a further aspect, a method of detecting the presence of LAG3 in a biological sample is provided. In certain embodiments, the method comprises contacting the biological sample with an anti-LAG3 antibody as described herein under conditions permissive for binding of the anti-LAG3 antibody to LAG3, and detecting whether a complex is formed between the anti-LAG3 antibody and LAG3. Such method may be an *in vitro* or *in vivo* method. In one embodiment, an anti-LAG3 antibody is used to select subjects eligible for therapy with an anti-LAG3 antibody, e.g. where LAG3 is a biomarker for selection of patients.

Exemplary disorders that may be diagnosed using an antibody of the invention include cancer in different forms such as Chronic lymphocytic leukemia (CLL) breast cancer etc (see also the list of cancers below)

In certain embodiments, labeled anti-LAG3 antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

### E. Pharmaceutical Formulations

Pharmaceutical formulations of an anti-LAG3 antibody as described herein are prepared by mixing such antibody having the desired degree of purity with one or more optional pharmaceutically acceptable carriers (*Remington's Pharmaceutical Sciences* 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide anti-PD1 or anti PDL1 antibodies, or anti TIM3 antibodies. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in *Remington's Pharmaceutical Sciences* 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, *e.g.* films, or microcapsules.

The formulations to be used for *in vivo* administration are generally sterile. Sterility may be readily accomplished, e.g., by filtration through sterile filtration membranes.

### F. Therapeutic Methods and Compositions

Any of the anti-LAG3 antibodies provided herein may be used in therapeutic methods.

In one aspect, an anti-LAG3 antibody for use as a medicament is provided. In further aspects, an anti-LAG3 antibody or use in treating cancer is provided. In certain embodiments, an anti-LAG3 antibody for use in a method of treatment is provided. In certain embodiments, the invention provides an anti-LAG3 antibody for use in a method of treating an individual having cancer comprising administering to the individual an effective amount of the anti-LAG3 antibody. In one embodiment the antibody is for use in treating or delaying progression of an immune related disease such as tumor immunity. In one embodiment the antibody is for use in stimulating an immune response or function, such as T cell activity.

In further embodiments, the invention provides an anti-LAG3 antibody for use as immunostimmulatory agent/ or stimulating granzyme B (GrzB), interferon-gamma (IFN-gamma) and or interleukin 2 (IL-2) secretion/release. In certain embodiments, the invention provides an anti-LAG3 antibody for use in a method of granzyme B (GrzB), interferon-gamma (IFN-gamma) and or interleukin 2 (IL-2) secretion/release in an individual comprising administering to the individual an effective of the the anti-LAG3 antibody for granzyme B (GrzB), interferon-gamma (IFN-gamma) and or interleukin 2 (IL-2) secretion/release.

An "individual" according to any of the above embodiments is preferably a human. In a further aspect, the invention provides for the use of an anti-LAG3 antibody in the manufacture or preparation of a medicament. In one embodiment, the medicament is for treatment of cancer. In a further embodiment, the medicament is for use in a method of treating cancer comprising administering to an individual having cancer an effective amount of the medicament. In a further embodiment, the medicament is for inducing cell mediated lysis of cancer cells In a further embodiment, the medicament is for use in a method of inducing cell mediated lysis of cancer cells in an individual suffering from cancer comprising administering to the individual an amount effective of the medicament to induce apoptosis in a cancer cell/ or to inhibit cancer cell proliferation. An "individual" according to any of the above embodiments may be a human.

The term "cancer" as used herein may be, for example, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. In one preferred embodiment such cancer is a breast cancer, colorectal cancer, melanoma, head and neck cancer, lung cancer or prostate cancer. In one preferred embodiment such cancer is a breast cancer, ovarian cancer, cervical cancer, lung cancer or prostate cancer. In another preferred embodiment such cancer is breast cancer, lung cancer, colon cancer, ovarian cancer, melanoma cancer, bladder cancer, renal cancer, kidney cancer, liver cancer, head and neck cancer, colorectal cancer, pancreatic cancer, gastric carcinoma cancer, esophageal cancer, mesothelioma, prostate cancer, leukemia, lymphoma, myelomas. In one preferred embodiment such cancers are further characterized by LAG3 expression or overexpression.

In a further aspect, the invention provides pharmaceutical formulations comprising any of the anti-LAG3 antibodies provided herein, e.g., for use in any of the above therapeutic methods. In one embodiment, a pharmaceutical formulation comprises any of the anti-LAG3 antibodies provided herein and a pharmaceutically acceptable carrier.

In a further aspect, the invention provides pharmaceutical formulations comprising any of the anti-LAG3 antibodies provided herein, e.g., for use in any of the above therapeutic methods. In one embodiment, a pharmaceutical formulation comprises any of the anti-LAG3 antibodies provided herein and a pharmaceutically acceptable carrier. In another embodiment, a pharmaceutical formulation comprises any of the anti-LAG3 antibodies provided herein and at least one additional therapeutic agent, e.g., as described below.

Antibodies of the invention can be used either alone or in combination with other agents in a therapy. For instance, an antibody of the invention may be co-administered with at least one additional therapeutic agent. In certain embodiments, an additional therapeutic agent is an anti-LAG3 or anti PDL1 or anti TIM3 antibody.

In addition to the anti-LAG3 antibody antibody also a chemotherapeutic agent can be administered. In one embodiment such additional chemotherapeutic agents, which may be administered with anti-LAG3 antibody as described herein and the, include, but are not limited to, anti-neoplastic agents including alkylating agents including: nitrogen mustards, such as mechlorethamine, cyclophosphamide, ifosfamide, melphalan and chlorambucil; nitrosoureas, such as carmustine (BCNU), lomustine (CCNU), and semustine (methyl-CCNU); Temodal(TM) (temozolamide), ethylenimines/methylmelamine such as thriethylenemelamine (TEM), triethylene, thiophosphoramide (thiotepa), hexamethylmelamine (HMM, altretamine); alkyl sulfonates such as busulfan; triazines such as dacarbazine (DTIC); antimetabolites including folic acid analogs such as methotrexate and trimetrexate, pyrimidine analogs such as 5-fluorouracil (5FU), fluorodeoxyuridine, gemcitabine, cytosine arabinoside (AraC, cytarabine), 5-azacytidine, 2,2'-difluorodeoxycytidine, purine analogs such as 6-merca.rho.topurine, 6-thioguamne, azathioprine, T-deoxycoformycin (pentostatin), erythrohydroxynonyladenine (EHNA), fludarabine phosphate, and 2- chlorodeoxyadenosine (cladribine, 2-CdA); natural products including antimitotic drugs such as paclitaxel, vinca alkaloids including vinblastine (VLB), vincristine, and vinorelbine, taxotere, estramustine, and estramustine phosphate; pipodophylotoxins such as etoposide and teniposide; antibiotics such as actinomycin D, daunomycin (rubidomycin), doxorubicin, mitoxantrone, idarubicin, bleomycins, plicamycin (mithramycin), mitomycin C, and actinomycin; enzymes such as L-asparaginase; biological response modifiers such as interferon-alpha, IL-2, G-CSF and GM-CSF; miscellaneous agents including platinum coordination complexes such as oxaliplatin, cisplatin and carboplatin, anthracenediones such as mitoxantrone, substituted urea such as hydroxyurea, methylhydrazine derivatives including N- methylhydrazine (MIH) and procarbazine, adrenocortical suppressants such as mitotane (o, p-DDD) and aminoglutethimide; hormones and antagonists including adrenocorticosteroid antagonists such as prednisone and equivalents, dexamethasone and aminoglutethimide; Gemzar(TM) (gemcitabine), progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide. Therapies targeting epigenetic mechanism including, but not limited to, histone deacetylase inhibitors, demethylating agents (e.g., Vidaza) and release of transcriptional repression (ATRA) therapies can also be combined with the antigen binding proteins. In one embodiment the chemotherapeutic agent is selected from the group consisting of taxanes (like e.g. paclitaxel (Taxol), docetaxel (Taxotere), modified paclitaxel (e.g., Abraxane and Opaxio), doxorubicin, sunitinib (Sutent), sorafenib (Nexavar), and other multikinase inhibitors, oxaliplatin, cisplatin and carboplatin, etoposide, gemcitabine, and vinblastine. In one embodiment the chemotherapeutic agent is selected from the group consisting of taxanes (like e.g. taxol (paclitaxel), docetaxel (Taxotere), modified paclitaxel (e.g. Abraxane and Opaxio). In one embodiment, the additional chemotherapeutic agent is selected from 5-fluorouracil (5-FU), leucovorin, irinotecan, or oxaliplatin. In one embodiment the chemotherapeutic agent is 5-fluorouracil, leucovorin and irinotecan (FOLFIRI). In one embodiment the chemotherapeutic agent is 5-fluorouracil, and oxaliplatin (FOLFOX).

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent or agents. In one embodiment, administration of the anti-LAG3 antibody and administration of an additional therapeutic agent occur within about one month, or within about one, two or three weeks, or within about one, two, three, four, five, or six days, of each other. Antibodies of the invention might also be used in combination with radiation therapy.

An antibody of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments. Depending on the type and severity of the disease, about 1 µg/kg to 15 mg/kg (e.g. 0.1mg/kg-10mg/kg) of antibody can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or *e.g.* about six doses of the antibody). An initial higher loading dose, followed by one or more lower doses may be administered. An exemplary dosing regimen comprises administering. However, other dosage regimens may be useful. The progress of this therapy is easily monitored by conventional techniques and assays.

It is understood that any of the above formulations or therapeutic methods may be carried out using an immunoconjugate of the invention in place of or in addition to an anti-LAG3 antibody.

### G. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an antibody of the invention. The label or package insert indicates that the composition is used for treating the condition of choice. Moreover, the article of manufacture may comprise (a) a first container with a composition contained therein, wherein the composition comprises an antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### Description of the amino acid sequences and nucleic acid sequences

| | |
|---|---|
| SEQ ID NO: 1 | heavy chain HVR-H1, aLAG3(0414) |
| SEQ ID NO: 2 | heavy chain HVR-H2, aLAG3 (0414) |
| SEQ ID NO: 3 | heavy chain HVR-H3, aLAG3 (0414) |
| SEQ ID NO: 4 | light chain HVR-L 1, aLAG3 (0414) |
| SEQ ID NO: 5 | light chain HVR-L2, aLAG3 (0414) |
| SEQ ID NO: 6 | light chain HVR-L3, aLAG3 (0414) |
| SEQ ID NO: 7 | heavy chain variable domain VH, aLAG3(0414) |
| SEQ ID NO: 8 | light chain variable domain VL, aLAG3(0414) |
| | |
| SEQ ID NO: 9 | heavy chain HVR-H1, aLAG3(0403) |
| SEQ ID NO: 10 | heavy chain HVR-H2, aLAG3(0403) |
| SEQ ID NO: 11 | heavy chain HVR-H3, aLAG3(0403) |
| SEQ ID NO: 12 | light chain HVR-L1, aLAG3(0403) |
| SEQ ID NO: 13 | light chain HVR-L2, aLAG3(0403) |
| SEQ ID NO: 14 | light chain HVR-L3, aLAG3(0403) |
| SEQ ID NO: 15 | heavy chain variable domain VH, aLAG3(0403) |
| SEQ ID NO: 16 | light chain variable domain VL, aLAG3(0403) |
| SEQ ID NO: 17 | heavy chain HVR-H1, aLAG3(0411) |
| SEQ ID NO: 18 | heavy chain HVR-H2, aLAG3(0411) |
| SEQ ID NO: 19 | heavy chain HVR-H3, aLAG3(0411) |
| SEQ ID NO: 20 | light chain HVR-L 1, aLAG3 (0411) |
| SEQ ID NO: 21 | light chain HVR-L2, aLAG3(0411) |
| SEQ ID NO: 22 | light chain HVR-L3, aLAG3(0411) |
| SEQ ID NO: 23 | heavy chain variable domain VH, aLAG3(0411) |
| SEQ ID NO: 24 | light chain variable domain VL, aLAG3(0411) |
| | |
| SEQ ID NO: 25 | heavy chain HVR-H1, aLAG3(0417) |
| SEQ ID NO: 26 | heavy chain HVR-H2, aLAG3(0417) |
| SEQ ID NO: 27 | heavy chain HVR-H3, aLAG3(0417) |
| SEQ ID NO: 28 | light chain HVR-L 1, aLAG3(0417) |
| SEQ ID NO: 29 | light chain HVR-L2, aLAG3(0417) |
| SEQ ID NO: 30 | light chain HVR-L3, aLAG3(0417) |
| SEQ ID NO: 31 | heavy chain variable domain VH, aLAG3(0417) |
| SEQ ID NO: 32 | light chain variable domain VL, aLAG3(0417) |
| | |
| SEQ ID NO: 33 | heavy chain HVR-H1, aLAG3(0416) |
| SEQ ID NO: 34 | heavy chain HVR-H2, aLAG3(0416) |
| SEQ ID NO: 35 | heavy chain HVR-H3, aLAG3(0416) |
| SEQ ID NO: 3 6 | light chain HVR-L 1, aLAG3 (0416) |
| SEQ ID NO: 37 | light chain HVR-L2, aLAG3(0416) |
| SEQ ID NO: 38 | light chain HVR-L3, aLAG3(0416) |
| SEQ ID NO: 39 | heavy chain variable domain VH, aLAG3(0416) |
| SEQ ID NO: 40 | light chain variable domain VL, aLAG3(0416) |
| SEQ ID NO: 41 | heavy chain variable domain VH, BMS-986016 (WO2014/008218 and US2016/0326248) |
| SEQ ID NO: 42 | light chain variable domain VL BMS-986016 (WO2014/008218 and US2016/0326248) |
| SEQ ID NO: 43 | heavy chain variable domain VH, MDX25F7 (25F7) (US2011/0150892 and WO2014/008218) |
| SEQ ID NO: 44 | light chain variable domain VL, MDX25F7 (25F7) (US2011/0150892 and WO2014/008218) |
| SEQ ID NO: 45 | heavy chain variable domain VH, humanized BAP050 (LAG525) (US2015/0259420) |
| SEQ ID NO: 46 | light chain variable domain VL, humanized BAP050 (LAG525) (US2015/0259420) |
| SEQ ID NO: 47 | heavy chain variable domain VH, MDX26H10 (26H10) (US 2011/0150892) |
| SEQ ID NO: 48 | light chain variable domain VL, MDX26H10 (26H10) (US 2011/0150892) |
| SEQ ID NO: 49 | human kappa light chain constant region |
| SEQ ID NO: 50 | human lambda light chain constant region |
| SEQ ID NO: 51 | human heavy chain constant region derived from IgG1 |
| SEQ ID NO: 52 | human heavy chain constant region derived from IgG1 with mutations L234A, L235A and P329G |
| SEQ ID NO: 53 | human heavy chain constant region derived from IgG4 |
| SEQ ID NO: 54 | exemplary human LAG3 sequence (without signal sequence) |
| SEQ ID NO: 55 | human LAG3 Extracellular Domain (ECD) |
| SEQ ID NO: 56 | primer rbHC.up |
| SEQ ID NO: 57 | primer rbHCf.do |
| SEQ ID NO: 58 | primer BcPCR FHLC leader.fw |
| SEQ ID NO: 59 | primer BcPCR_huCkappa.rev |

In the following the amino acid sequences of the VH und VL domains including marked HVRs (HVRs in bold, underlined letters) of anti-LAG3 antibodies are listed:
**1)** aLAG3(0403)
   SEQ ID NO 15: VH
   SEQ ID NO 16: VL
**2)** aLAG3(0411)
   SEQ ID NO 23: VH
   SEQ ID NO 24: VL
**3)** aLAG3(0414)
   SEQ ID NO 7: VH
   SEQ ID NO 8: VL
**4)** aLAG3(0416)
   SEQ ID NO 39: VH
   SEQ ID NO 40: VL
**5)** aLAG3(0417)
   SEQ ID NO 31: VH
   SEQ ID NO 32: VL

### III. EXAMPLES

The following are examples of methods and compositions of the invention. They are not to be construed as limiting the invention, which is defined by the appended claims.

### Example 1:

### Generation of anti-LAG3 antibodies

### Immunization of rabbits

Roche proprietary transgenic rabbits expressing a humanized antibody repertoire were immunized with LAG3 expressing plasmid DNA.

A set of 3 rabbits was immunized genetically, using a plasmid expression vector coding for full-length human LAG3 (*15352*_*pIntronA*_*fl-hLag3_DNA-IMS*), by intradermal application of 400 ug vector DNA, followed by Electroporation (5 square pulses of 750 V/cm, duration 10 ms, interval 1 s). Rabbits received 7 consecutive immunizations at days 0, 14, 28, 49, 70, 98 and 126. Blood (10% of estimated total blood volume) was taken at days 35, 77, 105 and 133. Serum was prepared, which was used for titer determination by ELISA (see below), and peripheral mononuclear cells were isolated, which were used as a source of antigen-specific B cells in the B cell cloning process below.

### Determination of serum titers (ELISA)

Human recombinant LAG3 protein was immobilized on a 96-well NUNC Maxisorp plate at 2 ug/ml, 100 ul/well, in PBS, followed by: blocking of the plate with 2% Crotein C in PBS, 200 ul/well; application of serial dilutions of antisera, in duplicates, in 0.5% Crotein C in PBS, 100 ul/well; detection with either (1) HRP-conjugated donkey anti-rabbit IgG antibody (Jackson Immunoresearch/Dianova 711-036-152; 1/16 000), or (2) HRP-conjugated rabbit anti-human IgG antibody (Pierce/Thermo Scientific 31423; 1/5000), or (3) biotinylated goat anti-human kappa antibody (Southern Biotech/Biozol 2063-08, 1/5 000) and streptavidin-HRP; each diluted in 0.5% Crotein C in PBS, 100 ul/well. For all steps, plates were incubated for 1 h at 37° C. Between all steps, plates were washed 3 times with 0.05% Tween 20 in PBS. Signal was developed by addition of BM Blue POD Substrate soluble (Roche), 100 ul/well; and stopped by addition of 1 M HCl, 100 ul/well.

Absorbance was read out at 450 nm, against 690 nm as reference. Titer was defined as dilution of antisera resulting in half-maximal signal.

### Isolation of rabbit peripheral blood mononuclear cells (PBMC)

Blood samples were taken of immunized transgenic rabbits. EDTA containing whole blood was diluted twofold with 1× PBS (PAA, Pasching, Austria) before density centrifugation using lympholyte mammal (Cedarlane Laboratories, Burlington, Ontario, Canada) according to the specifications of the manufacturer. The PBMCs were washed twice with 1x PBS.

### EL-4 B5 medium

RPMI 1640 (Pan Biotech, Aidenbach, Germany) supplemented with 10% FCS (Hyclone, Logan, UT, USA), 2 mM Glutamin, 1% penicillin/streptomycin solution (PAA, Pasching, Austria), 2 mM sodium pyruvate, 10 mM HEPES (PAN Biotech, Aidenbach, Germany) and 0,05 mM b-mercaptoethanole (Gibco, Paisley, Scotland) was used.

### Coating of plates with protein antigen

Sterile cell culture 6-well plates were coated with human LAG3 ECD conjugated to a human Fc part (2 µg/ml) in carbonate buffer (0,1 M sodium bicarbonate, 34 mM Disodiumhydrogencarbonate, pH 9,55) over night at 4°C. Plates were washed in sterile PBS three times before use.

### Depletion of cells

(a) Sterile 6-well plates (cell culture grade) covered with a confluent monolayer of CHO cells were used to deplete macrophages/monocytes through unspecific adhesion as well as unspecifically binding lymphocytes.
(b) Blank sterile 6-well plates (cell culture grade) were used to deplete macrophages and monocytes and other cells through unspecific adhesion.

Half of the PBMC sample was used for (a) and half for (b).

Each well was filled at maximum with 4 ml medium and up to 6×106 PBMCs from the immunized rabbit and allowed to bind for 1 h at 37 °C in the incubator. The cells in the supernatant (peripheral blood lymphocytes (PBLs)) were used for the antigen panning step.

### Enrichment of B cells on LAG3 antigen

### Protein Antigen

6-well tissue culture plates coated with LAG3-ECD-huFc protein were seeded with up to 6 × 10e6 PBLs per 4 ml medium from the depletion steps using the blank 6-well plate and allowed to bind for 1 h at 37 °C in the incubator. Non-adherent cells were removed by carefully washing the wells 1-2 times with 1× PBS. The remaining sticky cells were detached by trypsin for 10 min at 37 °C in the incubator. Trypsination was stopped with EL-4 B5 medium. The cells were kept on ice until the immune fluorescence staining.

### Cell surface antigen

6-well tissue culture plates covered with a monolayer of human LAG3-positive CHO cells were seeded with up to 6×106 PBLs per 4 ml medium from the depletion steps using the CHO-covered 6-well plate and allowed to bind for 1 h at 37 °C in the incubator. Non-adherent cells were removed by carefully washing the wells 1-2 times with 1× PBS. The remaining sticky cells were detached by trypsin for 10 min at 37 °C in the incubator. Trypsination was stopped with EL-4 B5 medium. The cells were kept on ice until the immune fluorescence staining.

### Immune fluorescence staining and Flow Cytometry

The anti-IgG FITC (AbD Serotec, Düsseldorf, Germany) and the anti-huCk PE (Dianova, , Hamburg, Germany) antibody was used for single cell sorting. For surface staining, cells from the depletion and enrichment step were incubated with the anti-IgG FITC and the anti-huCk PE antibody in PBS and incubated for 45 min in the dark at 4°C. After staining the PBMCs were washed two fold with ice cold PBS. Finally the PBMCs were resuspended in ice cold PBS and immediately subjected to the FACS analyses. Propidium iodide in a concentration of 5 µg/ml (BD Pharmingen, San Diego, CA, USA) was added prior to the FACS analyses to discriminate between dead and live cells.

A Becton Dickinson FACSAria equipped with a computer and the FACSDiva software (BD Biosciences, USA) were used for single cell sort.

### B-cell cultivation

The cultivation of the rabbit B cells was performed by a method described by Seeber et al. (S Seeber et al. PLoS One 9 (2), e86184. 2014 Feb 04). Briefly, single sorted rabbit B cells were incubated in 96-well plates with 200 µl/well EL-4 B5 medium containing Pansorbin Cells (1:100000) (Calbiochem (Merck), Darmstadt, Deutschland), 5% rabbit thymocyte supernatant (MicroCoat, Bernried, Germany) and gamma-irradiated murine EL-4 B5 thymoma cells (5 × 10e5 cells/well) for 7 days at 37 °C in the incubator. The supernatants of the B-cell cultivation were removed for screening and the remaining cells were harvested immediately and were frozen at - 80 °C in 100 µl RLT buffer (Qiagen, Hilden, Germany).

### Isolation of V-Domains of LAG3 antibodies

### PCR amplification of V-domains

Total RNA was prepared from B cells lysate (resuspended in RLT buffer - Qiagen - Cat. N° 79216) using the NucleoSpin 8/96 RNA kit (Macherey&Nagel; 740709.4, 740698) according to manufacturer's protocol. RNA was eluted with 60 µl RNase free water. 6µl of RNA was used to generate cDNA by reverse transcriptase reaction using the Superscript III First-Strand Synthesis SuperMix (Invitrogen 18080-400) and an oligo dT-primer according to the manufatures's instructions. All steps were performed on a Hamilton ML Star System. 4µl of cDNA were used to amplify the immunoglobulin heavy and light chain variable regions (VH and VL) with the AccuPrime Supermix (Invitrogen 12344-040) in a final volume of 50µl using the primers rbHC.up and rbHC.do for the heavy chain and BcPCR_FHLC_leader.fw and BcPCR_huCkappa.rev for the light chain (Table 1.1). All forward primers were specific for the signal peptide (of respectively VH and VL) whereas the reverse primers were specific for the constant regions (of respectively VH and VL). The PCR conditions for the RbVH were as follows: Hot start at 94°C for 5 min; 35 cycles of 20s at 94°C, 20s at 70°C, 45s at 68 °C, and a final extension at 68°C for 7 min. The PCR conditions for the HuVL were as follows: Hot start at 94°C for 5 min; 40 cycles of 20s at 94°C, 20s at 52°C, 45s at 68 °C, and a final extension at 68°C for 7 min.

**Table 1.1**

| | |
|---|---|
| SEQ ID NO: 56 rbHC.up | |
| | |
| SEQ ID NO: 57 rbHCf.do | CCATTGGTGAGGGTGCCCGAG |
| SEQ ID NO: 58 BcPCR_FHLC_leader .fw | ATGGACATGAGGGTCCCCGC |
| SEQ ID NO: 59 BcPCR_huCkappa.rev | GATTTCAACTGCTCATCAGATGGC |

8µl of 50µl PCR solution were loaded on a 48 E-Gel 2% (Invitrogen G8008-02). Positive PCR reactions were cleaned using the NucleoSpin Extract II kit (Macherey&Nagel; 740609250) according to manufacturer's protocol and eluted in 50µl elution buffer. All cleaning steps were performed on a Hamilton ML Starlet System.

### Recombinant expression of rabbit monoclonal bivalent antibodies

For recombinant expression of rabbit monoclonal bivalent antibodies, PCR-products coding for VH or VL were cloned as cDNA into expression vectors by the overhang cloning method (RS Haun et al., Biotechniques (1992) 13, 515-518; MZ Li et al., Nature Methods (2007) 4, 251-256). The expression vectors contained an expression cassette consisting of a 5' CMV promoter including intron A, and a 3' BGH poly adenylation sequence. In addition to the expression cassette, the plasmids contained a pUC18-derived origin of replication and a beta-lactamase gene conferring ampicillin resistance for plasmid amplification in *E.coli.* Three variants of the basic plasmid were used: one plasmid containing the rabbit IgG constant region designed to accept the VH regions while containing human kappa LC constant region to accept the VL regions.

Linearized expression plasmids coding for the kappa or gamma constant region and VL /VH inserts were amplified by PCR using overlapping primers.

Purified PCR products were incubated with T4 DNA-polymerase which generated single-strand overhangs. The reaction was stopped by dCTP addition.

In the next step, plasmid and insert were combined and incubated with recA which induced site specific recombination. The recombined plasmids were transformed into E.coli. The next day the grown colonies were picked and tested for correct recombined plasmid by plasmid preparation, restriction analysis and DNA-sequencing.

For antibody expression, the isolated HC and LC plasmids were transiently co-transfected into HEK293 cells and the supernatants were harvested after 1 week.

### Example 2:

### Characterization anti-LAG3 antibodies

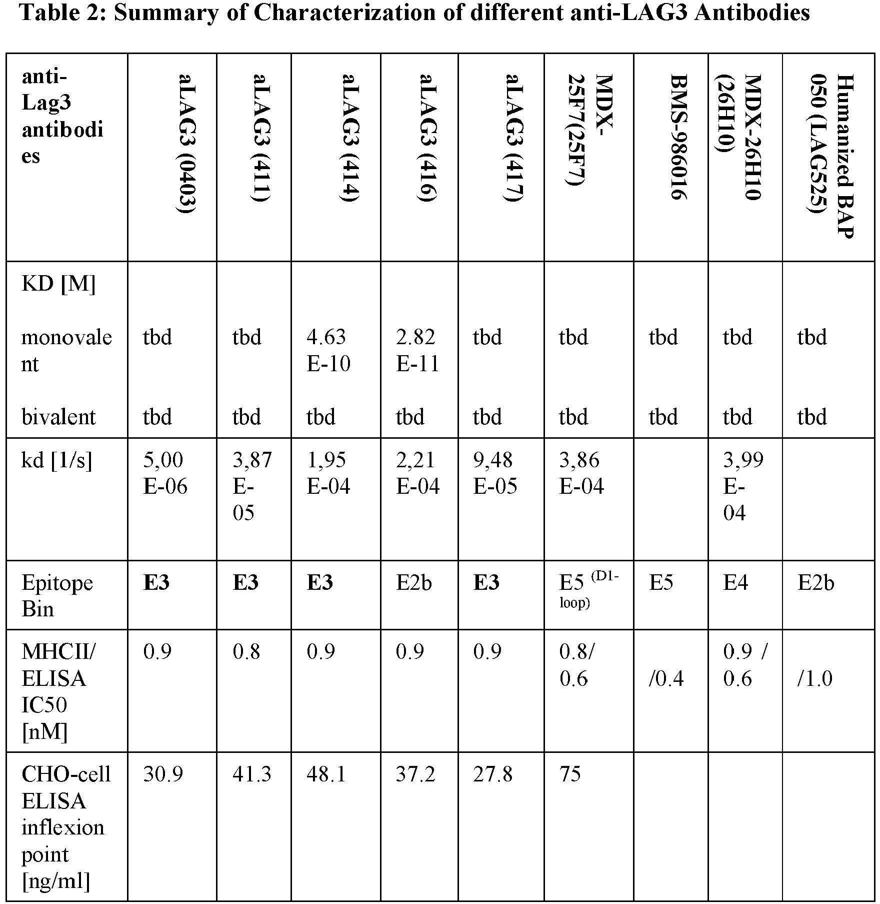

### ELISA for human Lag3

Nunc maxisorp plates (Nunc 464718) were coated with 25 µl/well recombinant Human LAG-3 Fc Chimera Protein (R&D Systems, 2319-L3) at a protein concentration of 800 ng/ml and incubated at 4°C overnight or for 1h at room temperature. After washing (3x90 µl/well with PBST-buffer) each well was incubated with 90 µl blocking buffer (PBS + 2% BSA + 0.05% Tween 20) for 1 h at room temperature. After washing (3x90 µl/well with PBST-buffer) 25 µl anti-Lag3 samples at a concentration of 1-9µg/ml (1:3 dilutions in OSEP buffer) were added and incubated 1h at RT. After washing (3x90 µl/well with PBST-buffer) 25µl/well goat anti-Human Ig κ chain antibody-HRP conjugate (Milipore, AP502P) was added in a 1:2000 dilution and incubated at RT for 1 h. After washing (3x90 µl/well with PBST-buffer) 25 µl/well TMB substrate (Roche, 11835033001) was added and incubated for 2-10 min. Measurement took place on a Tecan Safire 2 instrument at 370/492 nm.

### Cell-surface Lag3 binding ELISA

25 µl/well of Lag3 cells (recombinant CHO cells expressing Lag3, 10000 cells/well) were seeded into tissue culture treated 384-well plates (Corning, 3701) and incubated at 37°C for one or two days. The next day after removal of medium, 25 µl anti-Lag3 samples (1:3 dilutions in OSEP buffer, starting at a concentration of 6-40 nM) were added and incubated for 2h at 4°C. After washing (1 × 90µl in PBST) cells were fixed by addition of 30 µl/well glutaraldehyde to a final concentration of 0,05% (Sigma Cat.No: G5882), 10 min at room temperature. After washing (3x90 µl/well with PBST-buffer) 25µl/well goat anti-Human Ig κ chain antibody-HRP conjugate (Milipore, AP502P) was added in a 1:1000 dilution and incubated at RT for 1 h. After washing (3×90 µl/well with PBST-buffer) 25 µl/well TMB substrate (Roche, 11835033001) was added and incubated for 6- 10 min. Measurement took place on a Tecan Safire 2 instrument at 370/492 nm.

### SPR (Biacore) characterization of anti-LAG3 antibodies

A surface plasmon resonance (SPR) based assay has been used to determine the kinetic parameters of the binding between anti-Lag3 antibodies as monovalent Fab fragments and human Fc tagged human Lag3 extra cellular domains (ECDs) at 25°C.

Therefore two flow cells of a C1 biosensor chip were prepared in a Biacore T200 by immobilizing neutravidin, diluted to 25 µg/ml in acetate buffer pH 4.5, onto it using the 'immobilization wizard'. This yielded in immobilization levels of around 1900 RU. Then, CaptureSelect^{™} Biotin Anti-IgG-Fc (Human) Conjugate was bound to the neutravidin, using a 20 µg/ml dilution in running buffer (HBS-EP+, GE Healthcare)

The method itself consisted of four commands per cycle. First command: capturing of ~46 RU of huLag3-Fc (20s, 10 µl/min). Second command: sample injection for 120s followed by a 1200s long dissociation at a flow speed of 30 µl/min. Third and fourth command: regeneration by injecting Glycine-HCl pH 1.5 for 30 seconds.

A dilution series (3.13 nM - 200 nM, two-fold dilutions in running buffer) of each antibody Fab fragment and additional blank cycles were then measured using the previously described method. The Biacore T200 Evaluation Software was then utilized to gain kinetic values by applying a 1:1 langmuir fit with the Rmax fit parameter set to 'local' since the capture levels were not perfectly reproducible. Results are shown in table 2.

A surface plasmon resonance (SPR) based assay has been used to determine the apparent affinities of the interaction between aLag3 binders in their bivalent format and human Lag3 extra cellular domains (ECDs) at 25°C.

Therefore a Biacore biosensor chip was prepared in a Biacore T200, by immobilizing a minimum of about 800 RU of P329G point-mutation specific antibody, utilizing standard amine-coupling conditions.

Then, in each cycle, sample antibody was captured and one concentration of a huLag3 ECD concentration series (consisting of four concentrations in total) was applied to the system for 200s, followed by a 1200s long dissociation. The biosensor chip was then regenerated.

Resulting experimental data was evaluated using the 'Interaction Map' feature provided by Ridgeview Diagnostics TraceDrawer software, to calculate the individual apparent, affine binding contribution for each sample.

Results are shown in table 2.

### Epitope Mapping

Epitope binning was performed using a surface plasmon resonance (SPR) based assay. Therefore aLag3 binders were bound to huLag3 on a Biacore T200 instrument. Then the accessibility of other binders to the previously formed aLag3 binder - huLag3 complex was assessed.

A SA CAP Kit (GE Healthcare) was used to carry out this assay. If not described otherwise the assay was done according to the SA CAP Kit manual.

The run included only one cycle type. After hybridization, a 10 nM dilution of biotinylated, huFc-tagged huLag3 was allowed to bind to the streptavidin on the sensor chip for 20s at a flow rate of 10 µl/min. Then a first 200 nM sample diluted in running buffer was injected for 180s at a flow rate of 30 µl/min, immediately followed by a second sample under same conditions. The surface was then regenerated.

The samples were then assigned to different epitope groups with similar competition patterns. A first rough categorization was done, based on the relative response of the second injection using a threshold of 6.1 RU, which was just above the highest value observed when a binder was injected as first and second sample. All values and decisions were finally validated by visual inspection of the sensorgrams.

Results are shown in the table 2. Three major epitope patterns (E1, E2 and E3) were identified. Since aLag3-0416 and humanized BAP 050 share the same group but do not completely inhibit each other, they were assigned to subgroups E2b and E2c.

### Binding of anti-Lag3 antibodies from tg rabbits to recombinant cyno Lag3 positive HEK cells

In addition to the binding analysis using HEK cells recombinantely expressing human Lag3 on the surface, binding to cynomolgus Lag3-positive HEK cells was also evaluated. For this experiment, frozen HEK293F cells, previously transiently transfected with cyno-LAG-3, were thawed, centrifuged and resupplemented in PBS/2%FBS. 1.5×10⁵ cells/well were seeded into 96-well plates. Anti-Lag3 antibodies wered added to a final normalized concentration of 10µg/ml. For referencing and as controls, autofluorescence and positive control (Medarex 25F7) as well as isotype control (huIgG1 from Sigma, cat.no. # 15154, *data not shown*) antibodies were prepared and measured in the experiment. HEK cells were incubated with indicated antibodies for 45 min on ice, washed twice with 200µl ice-cold PBS buffercontaining 2% FBS, before secondary antibody (APC-labelled goat anti-human IgG-kappa, Invitrogen, cat.no.#MH10515) was added (1:50 diluted in FACS-Puffer/well) and further incubated for 30 min on ice. Cells were again washed twice with 200µl ice-cold PBS/2% FBS buffer before samples were finally resuspended in 150µl FACS buffer and binding was measured on FACS CANTO-II HTS Module.

### Results

Shown in the below table is the binding and cross-reactivtity of different anti-Lag3 antibodies to HEK293 cells expressing cynoLAG3, binding either gives in % positive cells or the GeoMean of the signal intensity:

| **LAG3 antibody** | **% pos.** | **GeoMean** |
|---|---|---|
| Reference LAG3 antibody MDX25F7 | 41.2 | 3062 |
| aLAG3(0411) | 88.6 | 11007 |
| aLAG3(0414) | 81.6 | 9169 |
| aLAG3(0416) | 67.9 | 4221 |
| aLAG3(0417) | 75.9 | 7115 |
| aLAG3(0403) | 82.0 | 7457 |

### Binding of anti-Lag3 antibodies from tg rabbits to (activated) cynomolgus PBMC/T cells expressing Lag3

After binding to recombinant Lag3 protein and Lag3 expressed recombinantly on mammalian cells, binding to Lag3 expressed on activated cynomolgus T cells was assessed/confirmed.

The binding characteristics of the newly generated anti-Lag3 antibodies (derived from Roche's transgenic rabbits) to Lag3 expressed on the cell surface of cynomolgus T cells or PBMC was confirmed by FACS analysis. While Lag3 is not expressed on naive T cells it is upregulated upon activation and/or on exhausted T cells. Thus, cynomolgus peripheral blood mononuclear cells (PBMC) were prepared from fresh cynomolgus blood and were then activated by CD3/CD28 pre-treatment (1µg/ml) for 2-3 days. Activated cells were subsequently analyzed for Lag3 expression: Briefly, 1-3×10⁵ activated cells were stained for 30-60 min on ice with indicated anti-Lag3 antibodies and respective control antibodies at 10µg/ml final concentration. The bound anti-Lag3 antibodies were detected via fluorochrome-conjugated anti-human IgG or an anti-rabbit IgG secondary antibodies. After staining, cells were washed two times with PBS/2% FCS and analyzed on a FACS Fortessa (BD).

### Results

The following table summarizes the percentage of Lag3 positive cells within activated cynomolgus PBMCs:

| **Anti-Lag3/ctrl Antibodies** | **% positive cyno cells (PBLs) after CD3/CD28 activation** |
|---|---|
| only 2nd Ab (hu) | 7.62 |
| DP47 (human isotype) | 9.19 |
| Reference LAG3 antibody (MDX25F7) | 22.1 |
| Reference LAG3 antibody BMS-986016 | 18.6 |
| Reference LAG3 antibody (humanized BAP050(LAG525)) | 50.7 |
| only 2nd Ab (rb) | 5.26 |
| aLAG3(0403) | 44.2 |
| aLAG3(0411) | 46.6 |
| aLAG3(0414) | 43.0 |
| aLAG3(0416) | 38.9 |
| aLAG3(0417) | 35.3 |

On activated cynomolgus T cells all of the rabbit anti-Lag3 antibodies demonstrated a significant binding to Lag3⁺ cells. Hereby, all newly generated antibodies showed an increased percentage of positive cells compared to human anti-Lag3 reference antibodies (e.g. such as MDX25F7, BMS-986016).

### Inhibition of LAG-3 binding to MHC-II expressed on human A375 tumor cells (by ELISA)

25 µl/well of A375 cells (10000 cells/well) were seeded into tissue culture treated 384-well plates (Corning, 3701) and incubated at 37°C overnight. Anti-Lag3 antibodies were pre-incubated for 1h with biotinylated-Lag3 (250 ng/ml) in cell culture medium in 1:3 dilutions starting at 3 µg/ml antibody-concentration. After removal of medium from the wells with the seeded cells, 25 µl of the antibody-Lag3 pre-incubated mixtures were transferred to the wells and incubated for 2 h at 4°C. After washing (1 × 90µl in PBST) cells were fixed by addition of 30 µl/well glutaraldehyde to a final concentration of 0,05% (Sigma Cat.No: G5882), 10 min at room temperature. After washing (3×90 µl/well with PBST-buffer) 25µl/well Poly-HRP40-Streptavidin (Fitzgerald, 65R-S104PHRPx) was added in a 1:2000 or 1:8000 dilution and incubated at RT for 1 h. After washing (3×90 µl/well with PB ST-buffer) 25 µl/well TMB substrate (Roche, 11835033001) was added and incubated for 2 to 10 min. Measurement took place on a Tecan Safire 2 instrument at 370/492 nm.

### Inhibition of LAG-3 binding to MHC-II expressed on human A375 tumor cells (by FACS analysis)

### Assay principle

To study the antagonistic function of the anti-Lag3 antibodies, an MHCII:Lag3 competition assay was conducted. MHCII⁺ human A375 cells were stained with inhouse generated biotinylated Lag3:Fc fusion protein with or without preincubation with anti-Lag3 antibodies. This analysis was studied in a FACS competition experiment: A375 cells (ATCC, #CRL-1619) were cultured for 2-3 passages in EM Eagle's medium supplemented with EBSS (PAN, cat.no. #P04-00509), 10% FBS, 2mM L-Glutamin, 1x NEAA and 1x Sodium Pyruvate. All antibodies, were diluted in FACS buffer to a final concentration of 20µg/ml in 25µl (in 96well U-bottom plates). 25µl of inhouse generated, biotinylated recombinant LAG-3:Fc fusion protein was added to a final concentration of 10µg/ml either to medium or to anti-Lag3 antibodies or controls and were pre-incubated for 30 min at room temperature. A375 cells were washed with PBS and adjusted to 3×10⁶ cells/ml in PBS. 100µl were seeded per well in a 96well V-bottom plate. Plates were centrifuged and supernatant was removed. Then the pre-incubated LAG-3:Fc fusion protein/ antibody mix (50µl/well) was added to the cells and incubated for 1h at room temperature. After this, cells were washed with 200µl FACS buffer. For detection of biotinylated Lag3:Fc protein bound to cellular MHCII, an APC-conjugated goat anti-Biotin antibody was used at 3µl/sample (Miltenyi Biotec, cat.no. #130-090-856) and incubated for additional 10-15 mins. After staining, cells were again washed and then transferred in 150µl FACS buffer (PBS/2% FBS) to a U-bottom plate and analyzed on a FACS Canto-II using an HTS module.

Two anti-Lag3 antibodies (clones 25F7 and 26H10; Medarex) served as positive controls and a human IgG1 (Sigma, cat.no. #15154) as appropriate isotype control. All antibodies were used at 10µg/ml final concentration.

### Results

Shown in the below table is the result of the FACS analysis demonstrating the percent inhibition of the Lag3 protein binding to MHC-II on cells (calculated as the reduced binding signal in reference to the maximal value in the absence of a blocking antibody):

| LAG3 antibody. | % Inhibition |
|---|---|
| aLAG3(0403) | 34.9 |
| aLAG3(0414) | 67.3 |
| aLAG3(0411) | 45.6 |
| aLAG3(0416) | 68.6 |
| aLAG3(0417) | 59.1 |
| Reference MDX25F7 | 70.0 |
| Reference MDX26H10 | 71.7 |
| Isotype control | -2.9 |
| No mAb | 0.0 |

These data support a functional interplay with Lag3 and blockade of the cellular interaction of all tested antibodies.

### Neutralizing potency of the novel anti-Lag3 antibodies in a standard LAG3 Blockade Bio/Reporterassay

To test the neutralizing potency of the novel anti-Lag3 antibodies in restoring a suppressed T cell response *in vitro,* a commercially available reporter system was used. This system consists of Lag3⁺ NFAT Jurkat effector cells (Promega, cat. no. #CS 194801), MHC-II⁺ Raji cells (ATCC, #CLL-86), and a super-antigen. In brief, the reporter system is based on three steps: (1) superantigen-induced NFAT cell activation, (2) inhibition of the activating signal mediated by the inhibiting interaction between MHCII (Raji cells) and Lag3⁺ NFAT Jurkat effector cells, and (3) recovery of the NFAT activation signal by Lag3-antagonistic/neutralizing aVH-Fc fusion constructs.

For this experiment, Raji and Lag-3⁺ Jurkat/NFAT-luc2 effector T cells were cultured as decribed by the provider. Serial dilutions (40pg/ml-50µg/ml) of several anti-Lag3 and reference antibodies were prepared in assay medium (RPMI 1640 (PAN Biotech, cat.no. #P04-18047), 1%FCS) in flat, white bottom 96-well culture plates (Costar, cat.no.#3917). 1×10⁵ Lag3⁺ NFAT-Jurkat cells/well) were added to the antibody solution. After this step, 2.5×10⁴ Raji cells/well were added to the Jurakt cell/antibody mix as well as 50ng/ml final concentration of the SED super- antigen (Toxin technology, cat.no. DT303). After an incubation of six hrs at 37°C and 5% CO₂, Bio-Glo substrate (Promega, #G7940) was warmed up to room temperature and 75 µl were added per well, incubated for 5-10 min before the overall luminescence was measured at a Tecan Infinite reader according to the kit's manufacturer's recommendation.

Shown in the diagrams is the restoration of a MHCII/Lag3-mediated suppression of the NFAT luciferase signal by different anti-Lag3 antibodies upon SED stimulation (given as EC50 values):

| **Anti-LAG3** | EC50 [nM] in Jurkat LAG3 + SED + Raji | | |
|---|---|---|---|
| | 1st assay | 2nd assay | 3rd assay |
| Reference MDX25F7 | 7.8/5.9 | 8.6 | n.t. |
| Reference BMS-986016 | n.t. | 9.6 | n.t. |
| Reference humanized BAP050(LAG525) | n.t. | 22.6 | n.t. |
| Lag3 IgG-Fc | n.t. | no effect | n.t. |
| aLAG3(0411) | 1.1 | 1.0 | n.t. |
| aLAG3(0414) | 1.1 | 1.0 | 1.8 |
| aLAG3(0416) | 3.1 | 2.5 | 3.5 |
| aLAG3(0417) | 1.0 | n.t. | n.t. |

n.t. molecules not tested in this experiment

### Example 3: Biological Activity in different assays: Effect of different anti-LAG3 Antibodies (alone or in combination with anti-PD1 antibodies)

**Table 3: Summary of Biologival activity of different anti-LAG3 Antibodies (alone or in combination with anit-PD1 antibodies)**

| **Assay type** | **Anti-Lag3 aLAG3 (0403)** | **Anti-Lag3 aLAG3 (0411)** | **Anti-Lag3 aLAG3 (0414)** | **Anti-Lag3 aLAG3 (0416)** | **Anti-Lag3 aLAG3 (0417)** | **Reference 1 BMS 986016** | **Reference 2 humanized BAP050 (LAG525)** |
|---|---|---|---|---|---|---|---|
| mMLR (GrzB) | + | - | +++ | ++ | + | - | ++ |
| mMLR (IL-2) | - | - | + | + | ++ | + | ++ |
| CD4+AR H77 | | | +++ | +++ | | + | + |
| Treg-suppression (GrzB) | | | +++ | + | | - | + |
| Treg-suppression (IFN-g) | | | +++ | ++ | | + | + |
| Melanoma patient PBMCs | | | +++ | | | | |

### Effect of PD-1 and LAG-3 blockade on cytotoxic Granzyme B release and IL-2 secretion by human CD4 T cells cocultured with allogeneic mature dendritic cells

To screen anti-LAG-3 blocking antibodies in combination with anti-PD-1 in an allogeneic setting we developed an assay in which freshly purified CD4 T cells are cocultured for 5 days in presence of monocyte-derived allogeneic mature dendritic cells (mDCs). Monocytes were isolated from fresh PBMCs one week before through plastic adherence followed by the removal of the non-adherent cells. We then generated immature DCs from the monocytes by culturing them for 5 days in media containing GM-CSF (50 ng/ml) and IL-4 (100 ng/ml). To induce iDCs maturation, we added TNF-alpha, IL-1beta and IL-6 (50 ng/ml each) to the culturing media for 2 additional days. We then assessed DCs maturation by measuring their surface expression of Major Histocompatibility Complex Class II (MHCII), CD80, CD83 and CD86 thorugh flow cytometry (LSRFortessa, BD Biosciences).

On the day of the minimal mixed lymphocyte reaction (mMLR), CD4 T cells were enriched via a microbead kit (Miltenyi Biotec) from 108 PBMCs obtained from an unrelated donor. Prior culture, CD4 T cells were labeled with 5µM of Carboxy-Fluorescein-Succinimidyl Esther (CFSE). 10⁵ CD4 T cells were then plated in a 96 well plate together with mature allo-DCs (5:1) in presence or absence of blocking anti-PD-1 antibody aPD1(0376) (= PD1-0103-0312, from PCT Application PCT/EP2016/073248) alone or in combination with chimeric anti-LAG-3 antibodies (aLAG3(0403) to aLAG(0418) ((0403) to (0418)) or reference antibodies (humanized BAP050 (LAG525) and BMS 986016) at the concentration of 10 µg/ml. DP47 is a non-binding human IgG with a LALA mutation in the Fc portion to avoid recognition by FcyR and was used as negative control.

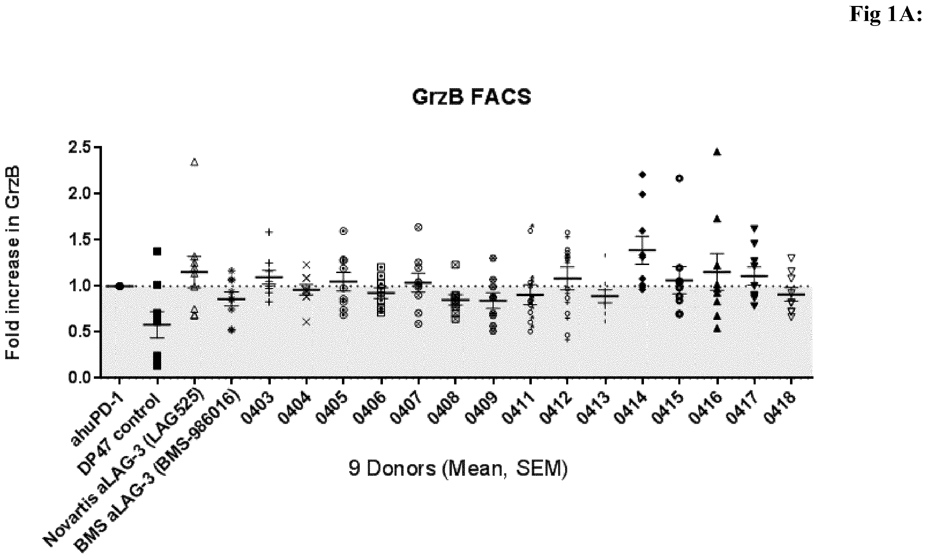

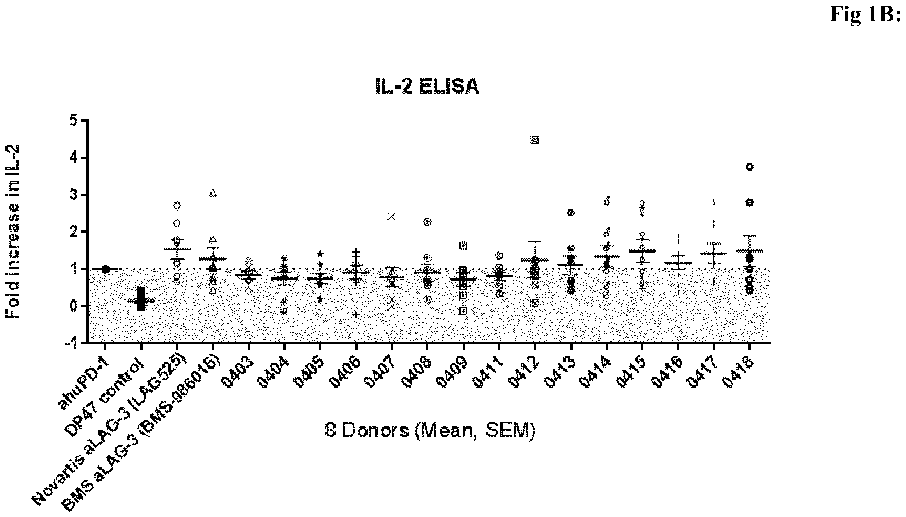

Five days later we collected the cell-culture supernatants, used later to measure the IL-2 levels by ELISA (R&D systems), and left the cells at 37 degree Celsius for additional 5 hours in presence of Golgi Plug (Brefeldin A) and Golgi Stop (Monensin). The cells were then washed, stained on the surface with anti-human CD4 antibody and the Live/Dead fixable dye Aqua (Invitrogen) before being fixed/permeabilized with Fix/Perm Buffer (BD Bioscience). We performed intracellular staining for Granzyme B (BD Bioscience) and IFN-γ (eBioscience). Results are shown in Figures 1A and B.

### Effect of PD-1 and LAG-3 blockade on cytotoxic Granzyme B release by human CD4 T cells cocultured with a B cell-lymphoblatoid cell line (ARH77).

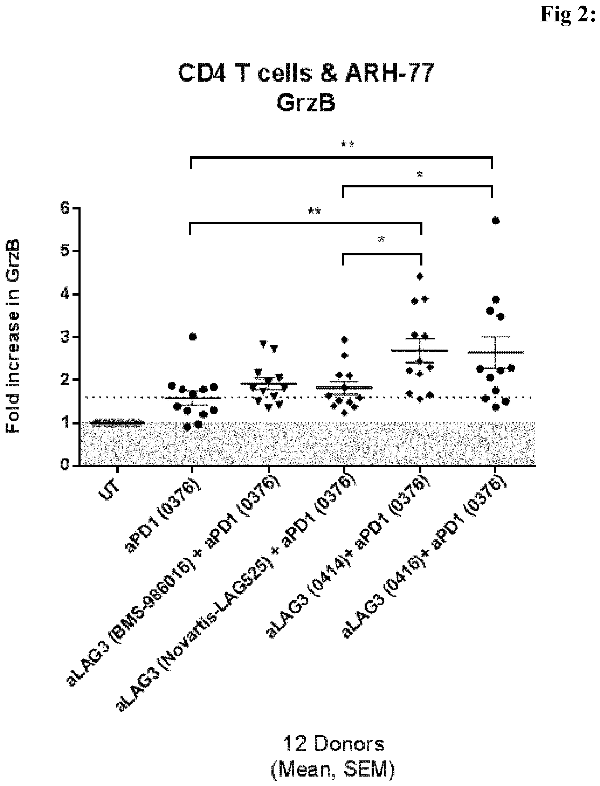

In functional studies, we co-cultured CD4 T cells with the tumor cell line ARH77, a B cell lymphoblastoid cell line which expresses lower levels of PDL-1 than mDCs, to better characterize the contribution of LAG-3 antagonism to PD-1 blockade. The rest of the experimental set up and readout remained unchanged from the mMLR. Our anti-LAG-3 antibodies (aLAG3(0414) and aLAG3(0416), chosen based on their ability to co-secrete IL-2 and Granzyme B in the mMLR) in combination with anti-PD-1 antibody showed a significant increase in Granzyme B secretion by CD4 T cells than reference anti-LAG-3 antibodies ((humanized BAP050 (LAG525) and BMS 986016) ) (P<0.05) and anti-PD-1 alone (P<0.01), Figure 2.

### Effect of PD-1 and LAG-3 blockade on Treg suppression of Granzyme B and IFN-γ release by human CD4 T cells cocultured with irradiated allogeneic PBMCs.

In functional studies involving regulatory T cells (Treg)-suppression assays, PBMCs from the same donor where divided in two samples: one was enriched in CD4 T cells and the other one in Tregs defined as CD4⁺CD25^{high} CD127^{low} T cells via a microbead kit (Miltenyi Biotec). Once purified the two populations, CD4 T cells were labelled with 5µM of Carboxy-Fluorescein-Succinimidyl Esther (CFSE) while Tregs with 5µM Cell-Trace-Violet (CTV) to be able to distinguish them at the FACS later on.

Both CD4 T cells (10⁵) and Tregs (10⁵) were then co-cultured in a 96 well plate at 1:1 ratio together with irradiated PBMCs (10⁵) from an unrelated donor in presence or absence of our anti-LAG-3 antibodies (aLAG3(0414) and aLAG3(0416) or reference anti-LAG-3 antibodies (humanized BAP050 (LAG525) and BMS 986016) in combination with our anti-PD-1 antibody at the concentration of 10 µg/ml. As control to estimate the magnitude of the suppression of CD4 T cell effector functions by Tregs, CD4 T cells (10⁵) were also co-cultured with irradiated PBMCs (10⁵) in the absence of Tregs.

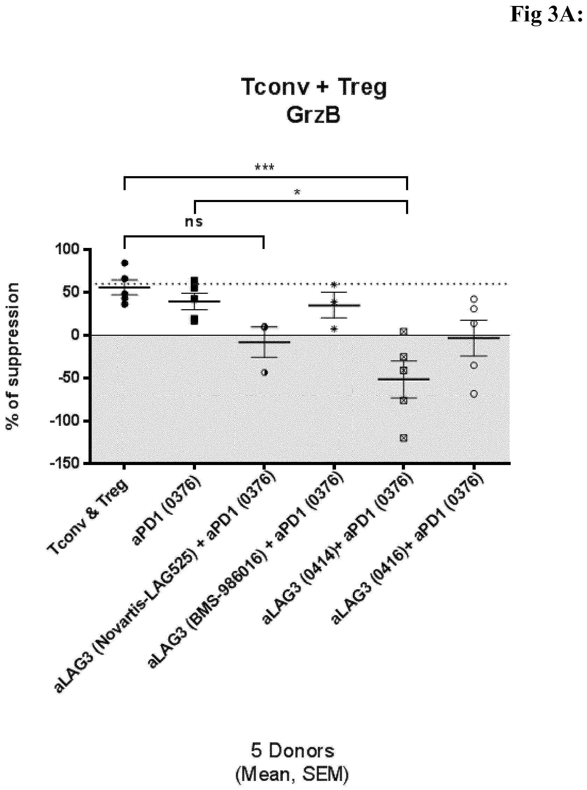

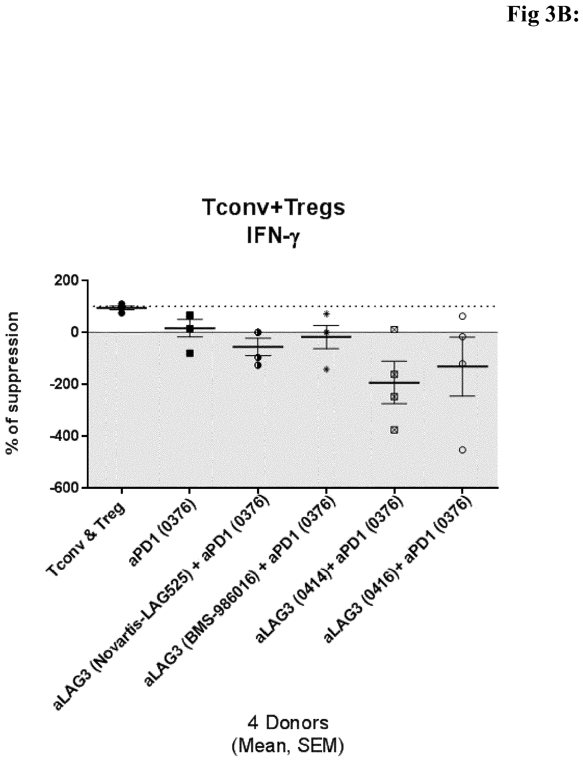

Five days later we collected the cell-culture supernatants, used later to measure IFN-γ levels by ELISA (R&D systems), and left the cells at 37 degrees Celsius for additional 5 hours in presence of Golgi Plug (Brefeldin A) and Golgi Stop (Monensin). The cells were then washed, stained on the surface with anti-human CD4 antibody and the Live/Dead fixable dye Aqua (Invitrogen) before being fixed/permeabilized with Fix/Perm Buffer (BD Bioscience). We performed intracellular staining for Granzyme B (BD Bioscience) and IFN-γ (eBioscience). Results are shown in Figures 3A and B.

The anti-LAG-3 antibodies (aLAG3(0414) and aLAG3(0416), in combination with anti-PD-1 antibody aPD1(0376) (= PD1-0103-0312, from PCT Application PCT/EP2016/073248) elicited Tconv escape from regulatory T cell tight control as demonstrated by the secretion of significantly higher amount of Granzyme B than Tconv in presence of anti-PD-1 alone (P<0.05) or in absence of checkpoint inhibitors (P<0.001). Reference anti-LAG-3 antibodies (humanized BAP050 (LAG525) and BMS 986016) in combination with anti-PD-1 did not significantly rescue Tconv effector functions from Treg suppression. Similar results were obtained for IFN-g even if the difference did not reach statistical significance with only 4 donors.

### Effect of PD-1 and LAG-3 blockade on Granzyme B and IFN-gamma secretion by CD4 T cells from melanoma patient PBMCs after recall with immunogenic melanoma-antigen peptide pools.

It has been previously described that melanoma patient PBMCs contain detectable frequencies of tumor-antigen specific T cells. Therefore, for POC purposes, we tested anti-LAG-3 antibody (0414) plus anti-PD-1 versus or anti-PD-1 alone on melanoma patient PBMCs re-stimulated overnight with immunogenic melanoma associated antigens peptide pools.

10⁵ to 10⁶ PBMCs from melanoma patients where incubated at room temperature in presence or absence of saturating concentrations (10 µg/ml) of anti-PD-1 alone (0376), in combination with anti-LAG-3 (aLAG3(0414) = (0414), 10 µg/ml) antibody. T cells were then re-stimulated over-night with a pool of immunogenic tumor related antigens like MAGEA1, MAGEA3, MAGEA4, Melan-A/MART-1, NYESO-1, Melanocyte protein Pmel 17 gp100, Tyrosinase, Tyrosinase-related protein 2 in presence of protein transport inhibitors Golgi Plug (Brefeldin A) and Golgi Stop (Monensin).

The cells were then washed, stained on the surface with anti-human CD4 antibody and the Live/Dead fixable dye Aqua (Invitrogen) before being fixed/permeabilized with Fix/Perm Buffer (BD Bioscience). We performed intracellular staining for Granzyme B (BD Bioscience) and IFN-γ (eBioscience).

The combination of anti-LAG-3 and anti-PD-1 antibodies (P<0.01 and *P*<0.001) significantly (P<0.01 and *P*<0.0001) enhanced tumor-antigen specific T cell effector functions (i.e. Granzyme B and IFN-γ secretion) while PD-1 blockade alone did not show any effect (data not shown).

Analogously one skilled in the art could extend the above under example 3 cell culture/animal studies to human methods of treatment.

## Claims

1. An isolated antibody that binds to human LAG3, wherein the antibody comprises
(a) a VH domain comprising (i) HVR-H1 comprising the amino acid sequence of SEQ ID NO:1, (ii) HVR-H2 comprising the amino acid sequence of SEQ ID NO:2, and (iii) HVR-H3 comprising an amino acid sequence selected from SEQ ID NO:3; and (b) a VL domain comprising (i) HVR-L1 comprising the amino acid sequence of SEQ ID NO:4; (ii) HVR-L2 comprising the amino acid sequence of SEQ ID NO:5 and (iii) HVR-L3 comprising the amino acid sequence of SEQ ID NO:6.

2. The antibody according to claim 1, wherein the antibody comprises a VH sequence of SEQ ID NO:7 and a VL sequence of SEQ ID NO:8.

3. The antibody according to anyone of claims 1 or 2, wherein the antibody:
i) competes for binding to LAG3 with an anti-LAG3 antibody comprising the VH with the amino acid sequence of SEQ ID NO:7 and VL with the amino acid sequence of SEQ ID NO:8, and/ or
ii) binds to a human and cynomolguoes LAG3; and/ or
iii) inhibits binding of MHC-II expressed on human A375 tumor cells; and/ or
iv) enhances granzyme B or IL-2 release in a mixed lymphocyte reaction (mMLR) assay.

4. The antibody according to any of the preceding claims, which is a human, humanized, or chimeric antibody.

5. The antibody of according to any one of the preceding claims, which is a full length IgG1 antibody with mutations L234A, L235A and P329G (numbering according to the EU index of Kabat).

6. Isolated nucleic acid encoding the antibody according to any one of the preceding claims.

7. A host cell comprising the nucleic acid of claim 6.

8. A method of producing an antibody comprising culturing the host cell of claim 7 so that the antibody is produced.

9. The method of claim 8; further comprising recovering the antibody from the host cell.

10. A pharmaceutical formulation comprising the antibody according any one of claims 1 to 5 and a pharmaceutically acceptable carrier.

11. The antibody according any one of claims 1 to 5 for use in treating cancer.

## Patentansprüche

1. Isolierter Antikörper, der an humanes LAG3 bindet, wobei der Antikörper Folgendes umfasst
(a) eine VH-Domäne, umfassend (i) HVR-H1, umfassend die Aminosäuresequenz von SEQ ID NO:1, (ii) HVR-H2, umfassend die Aminosäuresequenz von SEQ ID NO:2, und (iii) HVR-H3, umfassend eine Aminosäuresequenz, ausgewählt aus SEQ ID NO:3; und (b) eine VL-Domäne, umfassend (i) HVR-L1, umfassend die Aminosäuresequenz von SEQ ID NO:4; (ii) HVR-L2, umfassend die Aminosäuresequenz von SEQ ID NO:5, und (iii) HVR-L3, umfassend die Aminosäuresequenz von SEQ ID NO:6.

2. Antikörper nach Anspruch 1, wobei der Antikörper
eine VH-Sequenz von SEQ ID NO:7 und eine VL-Sequenz von SEQ ID NO:8 umfasst.

3. Antikörper nach einen der Ansprüche 1 oder 2, wobei der Antikörper:
i) mit einem Anti-LAG3-Antikörper, umfassend die VH mit der Aminosäuresequenz von SEQ ID NO:7 und VL mit der Aminosäuresequenz von SEQ ID NO:8, um das Binden an LAG3 konkurriert, und/oder
ii) an ein humanes und Javaneraffen-LAG3 bindet und/oder
iii) das Binden von MHC-II, der auf humanen A375-Tumorzellen exprimiert wird, inhibiert; und/oder
iv) Granzym-B- oder IL-2-Freisetzung in einem Mischlymphozytenreaktion (mMLR)-Assay verstärkt.

4. Antikörper nach einem der vorstehenden Ansprüche, der ein humaner, humanisierter oder chimärer Antikörper ist.

5. Antikörper nach einem der vorstehenden Ansprüche, der ein IgG1-Antikörper voller Länge mit Mutationen L234A, L235A und P329G (Nummerierung gemäß dem EU-Index von Kabat) ist.

6. Isolierte Nukleinsäure, die für den Antikörper nach einem der vorstehenden Ansprüche kodiert.

7. Wirtszelle, umfassend die Nukleinsäure nach Anspruch 6.

8. Verfahren zum Produzieren eines Antikörpers, umfassend das Kultivieren der Wirtszelle nach Anspruch 7, so dass der Antikörper produziert wird.

9. Verfahren nach Anspruch 8, ferner umfassend das Gewinnen des Antikörpers aus der Wirtszelle.

10. Pharmazeutische Formulierung, umfassend den Antikörper nach einem der Ansprüche 1 bis 5 und einen pharmazeutisch unbedenklichen Träger.

11. Antikörper nach einem der Ansprüche 1 bis 5 zur Verwendung bei der Behandlung von Krebs.

## Revendications

1. Anticorps isolé qui se lie au LAG3 humain, dans lequel l'anticorps comprend
(a) un domaine VH comprenant (i) une HVR-H1 comprenant la séquence d'acides aminés de SEQ ID NO:1, (ii) une HVR-H2 comprenant la séquence d'acides aminés de SEQ ID NO:2 et (iii) une HVR-H3 comprenant une séquence d'acides aminés choisie parmi SEQ ID NO:3 ; et (b) un domaine VL comprenant (i) une HVR-L1 comprenant la séquence d'acides aminés de SEQ ID NO:4; (ii) une HVR-L2 comprenant la séquence d'acides aminés de SEQ ID NO:5 et (iii) une HVR-L3 comprenant la séquence d'acides aminés de SEQ ID NO:6.

2. Anticorps selon la revendication 1, dans lequel l'anticorps
comprend une séquence VH de SEQ ID NO:7 et une séquence VL de SEQ ID NO:8.

3. Anticorps selon l'une quelconque des revendications 1 ou 2, dans lequel l'anticorps :
i) est en compétition pour la liaison au LAG3 avec un anticorps anti-LAG3 comprenant le VH avec la séquence d'acides aminés de SEQ ID NO:7 et un VL avec la séquence d'acides aminés de SEQ ID NO : 8, et/ou
ii) se lie à un LAG3 humain et de macaque crabier ; et/ou
iii) inhibe la liaison de CMH-II exprimé sur les cellules tumorales A375 humaines ; et/ou
iv) amplifie la libération de granzyme B ou d'IL-2 dans un dosage de réaction lymphocytaire mixte (mMLR).

4. Anticorps selon l'une quelconque des revendications précédentes, qui est un anticorps humain, humanisé ou chimérique.

5. Anticorps selon l'une quelconque des revendications précédentes, qui est un anticorps IgG1 pleine longueur avec les mutations L234A, L235A et P329G (numérotation selon l'indice EU de Kabat).

6. Acide nucléique isolé codant pour l'anticorps selon l'une quelconque des revendications précédentes.

7. Cellule hôte comprenant l'acide nucléique selon la revendication 6.

8. Procédé de production d'un anticorps comprenant la culture de la cellule hôte selon la revendication 7 de manière à produire l'anticorps.

9. Procédé selon la revendication 8; comprenant en outre la récupération de l'anticorps de la cellule hôte.

10. Formulation pharmaceutique comprenant l'anticorps selon l'une quelconque des revendications 1 à 5 et un véhicule pharmaceutiquement acceptable.

11. Anticorps selon l'une quelconque des revendications 1 à 5 pour une utilisation dans le traitement d'un cancer.


### SEQUENCE LISTING

<110> F. Hoffmann-La Roche AG
<120> ANTI-LAG3 ANTIBODIES
<130> P34228
<150> EP17164917.1
   <151> 2017-04-05
<160> 59
<170> PatentIn version 3.5
<210> 1
   <211> 5
   <212> PRT
   <213> homo sapiens
<400> 1
   Asp Tyr Thr Met Asn
1 5
<210> 2
   <211> 17
   <212> PRT
   <213> homo sapiens
<400> 2 Gly
<210> 3
   <211> 12
   <212> PRT
   <213> homo sapiens
<400> 3
   Gly Leu Thr Asp Thr Thr Leu Tyr Gly Ser Asp Tyr
   1 5 10
<210> 4
   <211> 11
   <212> PRT
   <213> homo sapiens
<400> 4
   Arg Ala Ser Gln Ser Ile Ser Ser Tyr Leu Asn
   1 5 10
<210> 5
   <211> 7
   <212> PRT
   <213> homo sapiens
<400> 5
   Ala Ala Ser Thr Leu Gln Ser
   1               5
<210> 6
   <211> 9
   <212> PRT
   <213> homo sapiens
<400> 6
   Gln Gln Thr Tyr Ser Ser Pro Leu Thr
   1               5
<210> 7
   <211> 121
   <212> PRT
   <213> homo sapiens
<400> 7
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Ile Phe Asp Asp Tyr
               20                  25                  30
   Thr Met Asn Trp Val Arg Gln Ala Pro Gly Lys Gly Leu Glu Trp Val
           35                  40                  45
   Ala Val Ile Ser Trp Asp Gly Gly Gly Thr Tyr Tyr Thr Asp Ser Val
       50                  55                  60
   Lys Gly Arg Phe Thr Ile Ser Arg Asp Asp Phe Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Leu Thr Asp Thr Thr Leu Tyr Gly Ser Asp Tyr Trp Gly
               100                 105                 110
   Gln Gly Thr Leu Val Thr Val Ser Ser
           115                 120
<210> 8
   <211> 107
   <212> PRT
   <213> homo sapiens
<400> 8
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr Ala Ala Ser Thr Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Thr Tyr Ser Ser Pro Leu
   85 90 95
   Thr Phe Gly Gly Gly Thr Lys Val Glu Ile Lys
   100 105
<210> 9
   <211> 5
   <212> PRT
   <213> homo sapiens
<400> 9
   Asp Tyr Thr Met His
   1 5
<210> 10
   <211> 17
   <212> PRT
   <213> homo sapiens
<400> 10
   Leu Val Ser Trp Asp Gly Gly Gly Thr Tyr Tyr Thr Asn Ser Val Lys
   1 5 10 15
   Gly
<210> 11
   <211> 12
   <212> PRT
   <213> homo sapiens
<400> 11
   Ala Ile Thr Asp Thr Ser Leu Tyr Gly Tyr Asp Tyr
   1 5 10
<210> 12
   <211> 11
   <212> PRT
   <213> homo sapiens
<400> 12
   Arg Ala Ser Gln Ser Ile Ser Ser Tyr Leu Asn
   1               5                   10
<210> 13
   <211> 7
   <212> PRT
   <213> homo sapiens
<400> 13
   Ala Ala Ser Ser Leu Gln Ser
   1               5
<210> 14
   <211> 9
   <212> PRT
   <213> homo sapiens
<400> 14
   Gln Gln Thr Tyr Ser Thr Pro Leu Thr
   1               5
<210> 15
   <211> 121
   <212> PRT
   <213> homo sapiens
<400> 15
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Asp Asp Tyr
               20                  25                  30
   Thr Met His Trp Val Arg Gln Ala Pro Gly Lys Gly Leu Glu Trp Val
           35                  40                  45
   Ser Leu Val Ser Trp Asp Gly Gly Gly Thr Tyr Tyr Thr Asn Ser Val
       50                  55                  60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Phe Cys
   85 90 95
   Ala Lys Ala Ile Thr Asp Thr Ser Leu Tyr Gly Tyr Asp Tyr Trp Gly
   100 105 110
   Gln Gly Ile Leu Val Thr Val Ser Ser
   115 120
<210> 16
   <211> 107
   <212> PRT
   <213> homo sapiens
<400> 16
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Asn Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Thr Tyr Ser Thr Pro Leu
   85 90 95
   Thr Phe Gly Gly Gly Thr Lys Val Glu Ile Lys
   100 105
<210> 17
   <211> 5
   <212> PRT
   <213> homo sapiens
<400> 17
   Asp Tyr Thr Met Asn
   1 5
<210> 18
   <211> 17
   <212> PRT
   <213> homo sapiens
<400> 18
   Val Ile Ser Trp Asp Gly Gly Ala Thr Tyr Tyr Ala Asp Ser Val Lys
   1 5 10 15
   Gly
<210> 19
   <211> 12
   <212> PRT
   <213> homo sapiens
<400> 19
   Gly Leu Thr Asp Asp Thr Leu Tyr Gly Ser Asp Tyr
   1 5 10
<210> 20
   <211> 11
   <212> PRT
   <213> homo sapiens
<400> 20
   Arg Ala Ser Gln Ser Ile Val Ser Tyr Leu Asn
   1 5 10
<210> 21
   <211> 7
   <212> PRT
   <213> homo sapiens
<400> 21
   Ala Ser Ser Ser Leu Gln Ser
   1 5
<210> 22
   <211> 9
   <212> PRT
   <213> homo sapiens
<400> 22
   Gln Gln Thr Tyr Ser Thr Pro Leu Thr
   1 5
<210> 23
   <211> 121
   <212> PRT
   <213> homo sapiens
<400> 23
   Glu Val His Leu Leu Glu Ser Gly Gly Gly Leu Val Gln Pro Gly Gly
   1               5                   10                  15
   Ser Leu Arg Leu Ser Cys Ala Ala Ser Gly Phe Ile Val Asp Asp Tyr
               20                  25                  30
   Thr Met Asn Trp Val Arg Gln Ala Pro Gly Lys Gly Leu Glu Trp Val
           35                  40                  45
   Ser Val Ile Ser Trp Asp Gly Gly Ala Thr Tyr Tyr Ala Asp Ser Val
       50                  55                  60
   Lys Gly Arg Phe Thr Ile Ser Arg Asp Asp Phe Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Leu Thr Asp Asp Thr Leu Tyr Gly Ser Asp Tyr Trp Gly
               100                 105                 110
   Gln Gly Thr Leu Val Thr Val Ser Ser
           115                 120
<210> 24
   <211> 107
   <212> PRT
   <213> homo sapiens
<400> 24
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Val Ser Tyr
               20                  25                  30
   Leu Asn Trp Tyr Gln Gln Lys Pro Gly Lys Ala Pro Lys Leu Leu Ile
           35                  40                  45
   Tyr Ala Ser Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Thr Tyr Ser Thr Pro Leu
                   85                  90                  95
   Thr Phe Gly Gly Gly Thr Lys Val Glu Ile Lys
               100                 105
<210> 25
   <211> 5
   <212> PRT
   <213> homo sapiens
<400> 25
   Asp Tyr Ala Met Ser
   1               5
<210> 26
   <211> 17
   <212> PRT
   <213> homo sapiens
<400> 26
   Gly Ile Asp Asn Ser Gly Tyr Tyr Thr Tyr Tyr Thr Asp Ser Val Lys
   1               5                   10                  15
   Gly
<210> 27
   <211> 13
   <212> PRT
   <213> homo sapiens
<400> 27
   Thr His Ser Gly Leu Ile Val Asn Asp Ala Phe Asp Ile
   1               5                   10
<210> 28
   <211> 11
   <212> PRT
   <213> homo sapiens
<400> 28
   Arg Ala Ser Gln Ser Ile Ser Ser Tyr Leu Asn
   1               5                   10
<210> 29
   <211> 7
   <212> PRT
   <213> homo sapiens
<400> 29
   Ala Ala Ser Ser Leu Gln Ser
   1               5
<210> 30
   <211> 9
   <212> PRT
   <213> homo sapiens
<400> 30
   Gln Gln Thr Tyr Ser Thr Pro Leu Thr
   1               5
<210> 31
   <211> 122
   <212> PRT
   <213> homo sapiens
<400> 31
   Glu Val Gln Leu Val Glu Ser Gly Gly Gly Leu Val Gln Pro Gly Gly
   1               5                   10                  15
   Ser Leu Arg Leu Ala Cys Ala Ala Ser Gly Phe Thr Phe Ser Asp Tyr
               20                  25                  30
   Ala Met Ser Trp Val Arg Gln Ala Pro Gly Lys Gly Leu Glu Trp Val
           35                  40                  45
   Ser Gly Ile Asp Asn Ser Gly Tyr Tyr Thr Tyr Tyr Thr Asp Ser Val
       50                  55                  60
   Lys Gly Arg Phe Thr Ile Ser Arg Asp Asp Val Lys Asn Thr Leu Tyr
   65                  70                  75                  80
   Leu Gln Met Asn Ser Leu Arg Ala Glu Asp Thr Ala Val Tyr Leu Cys
                   85                  90                  95
   Thr Lys Thr His Ser Gly Leu Ile Val Asn Asp Ala Phe Asp Ile Trp
               100                 105                 110
   Gly Gln Gly Thr Met Val Thr Val Ser Ser
           115                 120
<210> 32
   <211> 107
   <212> PRT
   <213> homo sapiens
<400> 32
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Thr Tyr Ser Thr Pro Leu
   85 90 95
   Thr Phe Gly Gly Gly Thr Lys Val Glu Ile Lys
   100 105
<210> 33
   <211> 5
   <212> PRT
   <213> homo sapiens
<400> 33
   Asp Tyr Ala Met Ser
   1 5
<210> 34
   <211> 17
   <212> PRT
   <213> homo sapiens
<400> 34
   Gly Ile Asp Asn Ser Gly Tyr Tyr Thr Tyr Tyr Thr Asp Ser Val Lys
   1 5 10 15
   Gly
<210> 35
   <211> 13
   <212> PRT
   <213> homo sapiens
<400> 35
   Thr His Ser Gly Leu Ile Val Asn Asp Ala Phe Asp Ile
   1 5 10
<210> 36
   <211> 11
   <212> PRT
   <213> homo sapiens
<400> 36
   Arg Ala Ser Gln Ser Ile Ser Ser Tyr Leu Asn
   1 5 10
<210> 37
   <211> 7
   <212> PRT
   <213> homo sapiens
<400> 37
   Asp Ala Ser Ser Leu Glu Ser
   1 5
<210> 38
   <211> 9
   <212> PRT
   <213> homo sapiens
<400> 38
   Gln Gln Ser Tyr Ser Thr Pro Leu Thr
   1 5
<210> 39
   <211> 122
   <212> PRT
   <213> homo sapiens
<400> 39
   Glu Val Gln Leu Val Glu Ser Gly Gly Gly Leu Val Gln Pro Gly Gly
   1 5 10 15
   Ser Leu Arg Leu Ala Cys Ala Ala Ser Gly Phe Thr Phe Ser Asp Tyr
   20 25 30
   Ala Met Ser Trp Val Arg Gln Ala Pro Gly Lys Gly Leu Glu Trp Val
   35 40 45
   Ser Gly Ile Asp Asn Ser Gly Tyr Tyr Thr Tyr Tyr Thr Asp Ser Val
   50 55 60
   Lys Gly Arg Phe Thr Ile Ser Arg Asp Asp Val Lys Asn Thr Leu Tyr
   65 70 75 80
   Leu Gln Met Asn Ser Leu Arg Ala Glu Asp Thr Ala Val Tyr Leu Cys
   85 90 95
   Thr Lys Thr His Ser Gly Leu Ile Val Asn Asp Ala Phe Asp Ile Trp
                           100                 105                 110
   Gly Gln Gly Thr Met Val Thr Val Ser Ser
           115                 120
<210> 40
   <211> 107
   <212> PRT
   <213> homo sapiens
<400> 40
   Asp Ile Gln Leu Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Asp Ala Ser Ser Leu Glu Ser Gly Val Pro Ser Arg Phe Ser Gly
       50                  55                  60
   Ser Gly Ser Gly Thr Asp Ala Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Leu
                   85                  90                  95
   Thr Phe Gly Gly Gly Thr Lys Val Glu Ile Lys
               100                 105
<210> 41
   <211> 120
   <212> PRT
   <213> Artificial
<220>
   <223> heavy chain variable domain VH, BMS-986016 (WO2014/008218 and US2016/0326248)
<400> 41
   Gln Val Gln Leu Gln Gln Trp Gly Ala Gly Leu Leu Lys Pro Ser Glu
   1 5 10 15
   Thr Leu Ser Leu Thr Cys Ala Val Tyr Gly Gly Ser Phe Ser Asp Tyr
   20 25 30
   Tyr Trp Asn Trp Ile Arg Gln Pro Pro Gly Lys Gly Leu Glu Trp Ile
   35 40 45
   Gly Glu Ile Asn His Arg Gly Ser Thr Asn Ser Asn Pro Ser Leu Lys
   50 55 60
   Ser Arg Val Thr Leu Ser Leu Asp Thr Ser Lys Asn Gln Phe Ser Leu
   65 70 75 80
   Lys Leu Arg Ser Val Thr Ala Ala Asp Thr Ala Val Tyr Tyr Cys Ala
   85 90 95
   Phe Gly Tyr Ser Asp Tyr Glu Tyr Asn Trp Phe Asp Pro Trp Gly Gln
   100 105 110
   Gly Thr Leu Val Thr Val Ser Ser
   115 120
<210> 42
   <211> 107
   <212> PRT
   <213> Artificial
<220>
   <223> light chain variable domain VL BMS-986016 (WO2014/008218 and US2016/0326248)
<400> 42
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Ile Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Leu
                   85                  90                  95
   Thr Phe Gly Gln Gly Thr Asn Leu Glu Ile Lys
               100                 105
<210> 43
   <211> 120
   <212> PRT
   <213> Artificial
<220>
   <223> heavy chain variable domain VH, MDX25F7 (25F7) (US2011/0150892 and WO2014/008218)
<400> 43
   Gln Val Gln Leu Gln Gln Trp Gly Ala Gly Leu Leu Lys Pro Ser Glu
   1               5                   10                  15
   Thr Leu Ser Leu Thr Cys Ala Val Tyr Gly Gly Ser Phe Ser Asp Tyr
               20                  25                  30
   Tyr Trp Asn Trp Ile Arg Gln Pro Pro Gly Lys Gly Leu Glu Trp Ile
           35                  40                  45
   Gly Glu Ile Asn His Asn Gly Asn Thr Asn Ser Asn Pro Ser Leu Lys
       50                  55                  60
   Ser Arg Val Thr Leu Ser Leu Asp Thr Ser Lys Asn Gln Phe Ser Leu
   65                  70                  75                  80
   Lys Leu Arg Ser Val Thr Ala Ala Asp Thr Ala Val Tyr Tyr Cys Ala
                   85                  90                  95
   Phe Gly Tyr Ser Asp Tyr Glu Tyr Asn Trp Phe Asp Pro Trp Gly Gln
               100                 105                 110
   Gly Thr Leu Val Thr Val Ser Ser
           115                 120
<210> 44
   <211> 107
   <212> PRT
   <213> Artificial
<220>
   <223> light chain variable domain VL, MDX25F7 (25F7) (US2011/0150892 and WO2014/008218)
<400> 44
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Ile Ser Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Leu
                   85                  90                  95
   Thr Phe Gly Gln Gly Thr Asn Leu Glu Ile Lys
               100                 105
<210> 45
   <211> 125
   <212> PRT
   <213> Artificial
<220>
   <223> heavy chain variable domain VH, humanized BAP050 (LAG525) (US2015/0259420)
<400> 45
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Phe Thr Leu Thr Asn Tyr
               20                  25                  30
   Gly Met Asn Trp Val Arg Gln Thr Pro Gly Lys Gly Leu Lys Trp Met
           35                  40                  45
   Gly Trp Ile Asn Thr Asp Thr Gly Glu Pro Thr Tyr Ala Asp Asp Phe
       50                  55                  60
   Lys Gly Arg Phe Ala Phe Ser Leu Glu Thr Ser Ala Ser Thr Ala Ser
   65                  70                  75                  80
   Leu Gln Ile Asn Asn Leu Lys Asn Ala Asp Thr Ala Thr Tyr Phe Cys
                   85                  90                  95
   Ala Arg Asn Pro Pro Tyr Tyr Tyr Gly Thr Asn Asn Ala Glu Ala Met
               100                 105                 110
   Asp Tyr Trp Gly Gln Gly Thr Thr Val Thr Val Ser Ser
           115                 120                 125
<210> 46
   <211> 107
   <212> PRT
   <213> Artificial
<220>
   <223> light chain variable domain VL, humanized BAP050 (LAG525) (US2015/0259420)
<400> 46
   Asp Ile Gln Met Thr Gln Thr Thr Ser Ser Leu Ser Ala Ser Leu Gly
   1               5                   10                  15
   Asp Arg Val Thr Ile Ser Cys Ser Ser Ser Gln Asp Ile Ser Asn Tyr
               20                  25                  30
   Leu Met Trp Tyr Gln Gln Lys Pro Asp Gly Thr Val Lys Val Leu Ile
           35                  40                  45
   Tyr Tyr Thr Ser Thr Leu His Leu Gly Val Pro Ser Arg Phe Ser Gly
       50                  55                  60
   Ser Gly Ser Gly Thr Asp Tyr Ser Leu Thr Ile Ser Asn Leu Glu Leu
   65                  70                  75                  80
   Glu Asp Ile Ala Thr Tyr Tyr Cys Gln Gln Tyr Tyr Asn Leu Pro Trp
                   85                  90                  95
   Thr Phe Gly Gln Gly Thr Lys Val Glu Ile Lys
               100                 105
<210> 47
   <211> 122
   <212> PRT
   <213> Artificial
<220>
   <223> heavy chain variable domain VH, MDX26H10 (26H10) (US 2011/0150892)
<400> 47
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Glu Trp Ala Val Ala Ser Trp Asp Tyr Gly Met Asp Val Trp
   100 105 110
   Gly Gln Gly Thr Thr Val Thr Val Ser Ser
   115 120
<210> 48
   <211> 108
   <212> PRT
   <213> Artificial
<220>
   <223> light chain variable domain VL, MDX26H10 (26H10) (US 2011/0150892)
<400> 48
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Pro
   85 90 95
   Phe Thr Phe Gly Pro Gly Thr Lys Val Asp Ile Lys
   100 105
<210> 49
   <211> 107
   <212> PRT
   <213> Homo Sapiens
<400> 49
<210> 50
   <211> 105
   <212> PRT
   <213> Homo Sapiens
<400> 50
   Gln Pro Lys Ala Ala Pro Ser Val Thr Leu Phe Pro Pro Ser Ser Glu
   1               5                   10                  15
   Glu Leu Gln Ala Asn Lys Ala Thr Leu Val Cys Leu Ile Ser Asp Phe
               20                  25                  30
   Tyr Pro Gly Ala Val Thr Val Ala Trp Lys Ala Asp Ser Ser Pro Val
           35                  40                  45
   Lys Ala Gly Val Glu Thr Thr Thr Pro Ser Lys Gln Ser Asn Asn Lys
       50                  55                  60
   Tyr Ala Ala Ser Ser Tyr Leu Ser Leu Thr Pro Glu Gln Trp Lys Ser
   65                  70                  75                  80
   His Arg Ser Tyr Ser Cys Gln Val Thr His Glu Gly Ser Thr Val Glu
                   85                  90                  95
   Lys Thr Val Ala Pro Thr Glu Cys Ser
               100                 105
<210> 51
   <211> 329
   <212> PRT
   <213> Homo Sapiens
<400> 51
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly
                   325
<210> 52
   <211> 329
   <212> PRT
   <213> homo sapiens
<400> 52
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
               115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Gly Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly
                   325
<210> 53
   <211> 326
   <212> PRT
   <213> Homo Sapiens
<400> 53
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Arg Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu Gly
   325
<210> 54
   <211> 497
   <212> PRT
   <213> homo sapiens
<400> 54
   Val Pro Val Val Trp Ala Gln Glu Gly Ala Pro Ala Gln Leu Pro Cys
   1 5 10 15
   Ser Pro Thr Ile Pro Leu Gln Asp Leu Ser Leu Leu Arg Arg Ala Gly
   20 25 30
   Val Thr Trp Gln His Gln Pro Asp Ser Gly Pro Pro Ala Ala Ala Pro
   35 40 45
   Gly His Pro Leu Ala Pro Gly Pro His Pro Ala Ala Pro Ser Ser Trp
   50 55 60
   Gly Pro Arg Pro Arg Arg Tyr Thr Val Leu Ser Val Gly Pro Gly Gly
   65 70 75 80
   Leu Arg Ser Gly Arg Leu Pro Leu Gln Pro Arg Val Gln Leu Asp Glu
   85 90 95
   Arg Gly Arg Gln Arg Gly Asp Phe Ser Leu Trp Leu Arg Pro Ala Arg
   100 105 110
   Arg Ala Asp Ala Gly Glu Tyr Arg Ala Ala Val His Leu Arg Asp Arg
   115 120 125
   Ala Leu Ser Cys Arg Leu Arg Leu Arg Leu Gly Gln Ala Ser Met Thr
   130 135 140
   Ala Ser Pro Pro Gly Ser Leu Arg Ala Ser Asp Trp Val Ile Leu Asn
   145 150 155 160
   Cys Ser Phe Ser Arg Pro Asp Arg Pro Ala Ser Val His Trp Phe Arg
   165 170 175
   Asn Arg Gly Gln Gly Arg Val Pro Val Arg Glu Ser Pro His His His
   180 185 190
   Leu Ala Glu Ser Phe Leu Phe Leu Pro Gln Val Ser Pro Met Asp Ser
   195 200 205
   Gly Pro Trp Gly Cys Ile Leu Thr Tyr Arg Asp Gly Phe Asn Val Ser
   210 215 220
   Ile Met Tyr Asn Leu Thr Val Leu Gly Leu Glu Pro Pro Thr Pro Leu
   225 230 235 240
   Thr Val Tyr Ala Gly Ala Gly Ser Arg Val Gly Leu Pro Cys Arg Leu
   245 250 255
   Pro Ala Gly Val Gly Thr Arg Ser Phe Leu Thr Ala Lys Trp Thr Pro
   260 265 270
   Pro Gly Gly Gly Pro Asp Leu Leu Val Thr Gly Asp Asn Gly Asp Phe
   275 280 285
   Thr Leu Arg Leu Glu Asp Val Ser Gln Ala Gln Ala Gly Thr Tyr Thr
   290 295 300
   Cys His Ile His Leu Gln Glu Gln Gln Leu Asn Ala Thr Val Thr Leu
   305 310 315 320
   Ala Ile Ile Thr Val Thr Pro Lys Ser Phe Gly Ser Pro Gly Ser Leu
   325 330 335
   Gly Lys Leu Leu Cys Glu Val Thr Pro Val Ser Gly Gln Glu Arg Phe
   340 345 350
   Val Trp Ser Ser Leu Asp Thr Pro Ser Gln Arg Ser Phe Ser Gly Pro
   355 360 365
   Trp Leu Glu Ala Gln Glu Ala Gln Leu Leu Ser Gln Pro Trp Gln Cys
   370 375 380
   Gln Leu Tyr Gln Gly Glu Arg Leu Leu Gly Ala Ala Val Tyr Phe Thr
   385 390 395 400
   Glu Leu Ser Ser Pro Gly Ala Gln Arg Ser Gly Arg Ala Pro Gly Ala
   405 410 415
   Leu Pro Ala Gly His Leu Leu Leu Phe Leu Ile Leu Gly Val Leu Ser
   420 425 430
   Leu Leu Leu Leu Val Thr Gly Ala Phe Gly Phe His Leu Trp Arg Arg
   435 440 445
   Gln Trp Arg Pro Arg Arg Phe Ser Ala Leu Glu Gln Gly Ile His Pro
   450 455 460
   Pro Gln Ala Gln Ser Lys Ile Glu Glu Leu Glu Gln Glu Pro Glu Pro
   465 470 475 480
   Glu Pro Glu Pro Glu Pro Glu Pro Glu Pro Glu Pro Glu Pro Glu Gln
   485 490 495
   Leu
<210> 55
   <211> 422
   <212> PRT
   <213> homo sapiens
<400> 55
   Val Pro Val Val Trp Ala Gln Glu Gly Ala Pro Ala Gln Leu Pro Cys
   1 5 10 15
   Ser Pro Thr Ile Pro Leu Gln Asp Leu Ser Leu Leu Arg Arg Ala Gly
   20 25 30
   Val Thr Trp Gln His Gln Pro Asp Ser Gly Pro Pro Ala Ala Ala Pro
   35 40 45
   Gly His Pro Leu Ala Pro Gly Pro His Pro Ala Ala Pro Ser Ser Trp
   50 55 60
   Gly Pro Arg Pro Arg Arg Tyr Thr Val Leu Ser Val Gly Pro Gly Gly
   65 70 75 80
   Leu Arg Ser Gly Arg Leu Pro Leu Gln Pro Arg Val Gln Leu Asp Glu
   85 90 95
   Arg Gly Arg Gln Arg Gly Asp Phe Ser Leu Trp Leu Arg Pro Ala Arg
   100 105 110
   Arg Ala Asp Ala Gly Glu Tyr Arg Ala Ala Val His Leu Arg Asp Arg
           115                 120                 125
   Ala Leu Ser Cys Arg Leu Arg Leu Arg Leu Gly Gln Ala Ser Met Thr
       130                 135                 140
   Ala Ser Pro Pro Gly Ser Leu Arg Ala Ser Asp Trp Val Ile Leu Asn
   145                 150                 155                 160
   Cys Ser Phe Ser Arg Pro Asp Arg Pro Ala Ser Val His Trp Phe Arg
                   165                 170                 175
   Asn Arg Gly Gln Gly Arg Val Pro Val Arg Glu Ser Pro His His His
               180                 185                 190
   Leu Ala Glu Ser Phe Leu Phe Leu Pro Gln Val Ser Pro Met Asp Ser
           195                 200                 205
   Gly Pro Trp Gly Cys Ile Leu Thr Tyr Arg Asp Gly Phe Asn Val Ser
       210                 215                 220
   Ile Met Tyr Asn Leu Thr Val Leu Gly Leu Glu Pro Pro Thr Pro Leu
   225                 230                 235                 240
   Thr Val Tyr Ala Gly Ala Gly Ser Arg Val Gly Leu Pro Cys Arg Leu
                   245                 250                 255
   Pro Ala Gly Val Gly Thr Arg Ser Phe Leu Thr Ala Lys Trp Thr Pro
               260                 265                 270
   Pro Gly Gly Gly Pro Asp Leu Leu Val Thr Gly Asp Asn Gly Asp Phe
           275                 280                 285
   Thr Leu Arg Leu Glu Asp Val Ser Gln Ala Gln Ala Gly Thr Tyr Thr
       290                 295                 300
   Cys His Ile His Leu Gln Glu Gln Gln Leu Asn Ala Thr Val Thr Leu
   305                 310                 315                 320
   Ala Ile Ile Thr Val Thr Pro Lys Ser Phe Gly Ser Pro Gly Ser Leu
                   325                 330                 335
   Gly Lys Leu Leu Cys Glu Val Thr Pro Val Ser Gly Gln Glu Arg Phe
               340                 345                 350
   Val Trp Ser Ser Leu Asp Thr Pro Ser Gln Arg Ser Phe Ser Gly Pro
           355                 360                 365
   Trp Leu Glu Ala Gln Glu Ala Gln Leu Leu Ser Gln Pro Trp Gln Cys
   370 375 380
   Gln Leu Tyr Gln Gly Glu Arg Leu Leu Gly Ala Ala Val Tyr Phe Thr
   385 390 395 400
   Glu Leu Ser Ser Pro Gly Ala Gln Arg Ser Gly Arg Ala Pro Gly Ala
   405 410 415
   Leu Pro Ala Gly His Leu
   420
<210> 56
   <211> 37
   <212> DNA
   <213> Artificial
<220>
   <223> primer rbHC.up
<400> 56
   aagcttgcca ccatggagac tgggctgcgc tggcttc 37
<210> 57
   <211> 21
   <212> DNA
   <213> Artificial
<220>
   <223> primer rbHCf.do
<400> 57
   ccattggtga gggtgcccga g 21
<210> 58
   <211> 20
   <212> DNA
   <213> Artificial
<220>
   <223> primer BcPCR_FHLC_leader.fw
<400> 58
   atggacatga gggtccccgc 20
<210> 59
   <211> 24
   <212> DNA
   <213> Artificial
<220>
   <223> primer BcPCR_huCkappa.rev
<400> 59
   gatttcaact gctcatcaga tggc 24